# EUROPÄISCHE PATENTSCHRIFT

(11) **EP 0 624 166 B1**
(45) Veröffentlichungstag und Bekanntmachung des Hinweises auf die Patenterteilung: **16.04.1997**
(21) Anmeldenummer: 94901820.4
(22) Anmeldetag: 19.11.1993
(51) Int. Cl.: C08B 37/16, C08B 31/04, C07H 13/04, C07H 13/08, C07H 19/16, C08F 20/10, G02B 1/04

(54) **POLYMERISIERBARE KOHLENHYDRATESTER, POLYMERE DARAUS UND DEREN VERWENDUNG**
POLYMERIZABLE CARBOHYDRATE ESTERS, POLYMERS MADE FROM SUCH ESTERS AND THE USE OF SUCH POLYMERS
ESTERS D'HYDRATE DE CARBONE POLYMERISABLES, POLYMERES AINSI OBTENUS ET LEUR UTILISATION

(30) Priorität: 30.11.1992 CH 3656/92
(43) Veröffentlichungstag der Anmeldung: 17.11.1994
(73) Patentinhaber: Novartis AG, 4058 Basel (CH)
(72) Erfinder: VETTER, Dirk, D-79110 Freiburg (DE)
(86) Internationale Anmeldenummer: EP9303236
(87) Internationale Veröffentlichungsnummer: WO9412540

(56) Entgegenhaltungen:
- GB-A- 1 134 235
- US-A- 3 565 887
- PATENT ABSTRACTS OF JAPAN vol. 14, no. 465 (P-1114)9. Oktober 1990 & JP,A,02 184 814 (SAKAKIBARA TOSHIYUKI) 19. Juli 1990
- MACROMOLECULES. Bd. 9, Nr. 5 , 1976 , EASTON US Seiten 701 - 704 A. HARADA ET AL. 'Cyclodextrin-containing polymers. 1. Preparation of polymers'

## Beschreibung

Die Erfindung betrifft polymerisierbare Ester aus Kohlenhydraten und Dicarbonsäuren mit einer radikalisch polymerisierbare Reste enthaltenden Estergruppe, Homopolymere und Copolymere daraus, Verfahren zu deren Herstellung und deren Verwendung.

Die Einführung von polymerisierbaren Gruppen in Kohlenhydrate wie zum Beispiel Cyclodextrine ist wegen deren Eigenschaften, besonders der hohen Hydrophilie, des spezifischen Komplexbildungsverhaltens und der Bioaktivität erwünscht. Acrylathaltige, methacrylathaltige und cinnamoylhaltige Cyclodextrine und Polymere daraus sind zum Beispiel von A. P. Croft et al. in Tetrahedron, Vol. 39, No. 9, Seiten 1425 bis 1427 (1983) beschrieben worden. Die polymerisierbaren Gruppen sind regiospezifisch in den 2- oder 3-Stellungen gebunden. Sie werden durch die Umsetzung von entsprechenden aktivierten Estern, nämlich Nitrophenylcarbonsäureestern mit einem Cyclodextrin erhalten. Das entstehende Nitrophenol kann im allgemeinen nur sehr schwer vollständig entfernt werden, da Cyclodextrine mit diesen organischen Verbindungen Einschlussverbindungen bilden. Wegen der physiologischen Bedenklichkeit von Nitrophenolen, deren polymerisationsinhibierende Wirkung und auch der sehr aufwendigen Reinigung können Polymere aus solchen polymerisierbaren Cyclodextrinen nur bedingt verwendet werden.

Es ist ferner bekannt, aus Acrylat- oder Methacrylatestern von Zuckern Homo- oder Copolymere herzustellen. Die Acylierung von Zuckern ist jedoch wenig regioselektiv und es werden stets uneinheitliche Verbindungsgemische erhalten, die auch Verbindungen mit mehr als einer polymerisierbaren Acylgruppe enthalten. Polymere aus diesen Gemischen enthalten daher stets unerwünschte und aus Lösungen zur Ausfällung neigende vernetzte oder verzweigte Produkte. Zudem gehen durch eine Mehrfachsubstitution die in vielen Anwendungen gewünschten natürlichen Eigenschaften der Zuckerreste verloren. Die Esterbindung dieser Acrylate und Methacrylate ist zudem relativ starr. Die Anwendungsbereiche werden durch diese Nachteile erheblich einschränkt. Um regioselektive substituierte Polymere von einem Acrylatester der Galaktose zu erhalten, ist vorgeschlagen worden, das Diacetonid von Galaktose zu acryloylieren, anschliessend zu polymerisieren und dann zu deblockieren (CA 70:29704p). Dieses aufwendige Verfahren ist jedoch unwirtschaftlich.

In der WO 91/17255 wird die mit Enzymen katalysierte Herstellung von Polymeren aus Zuckern und Dicarbonsäurestern über eine regioselektive Diacylierung beschrieben, wobei die Zuckerreste als Comonomere im Polymerrückgrat gebunden sind und dadurch bioaktive Eigenschaften praktisch verloren geht.

Es wurde nun gefunden, dass man Cyclosaccharide regioselektiv in den 2- oder 3-Stellungen monoacylieren oder die primäre Hydroxylgruppe von monomeren, dimeren oder linearen oligomeren Sacchariden regioselektiv monoacylieren kann, wenn man die Acylierung mit Mono- oder Divinylestern von Dicarbonsäuren durchführt. Man erhält überraschend trotz der Anwesenheit von zwei gleich reaktiven Estergruppen keine Dimeren oder mehrfach substituierte Produkte. Vinylestergruppen kann man mit ungesättigten Alkanolen zu anderen radikalisch polymerisierbaren Monomeren umestern oder mit ungesättigten Aminen amidieren. Diese Monomeren und die Monovinylester der Zucker eignen sich besonders zur Herstellung von linearen Polymerisaten, wobei die Saccharidgruppe an einen flexiblen Spacer gebunden ist. Durch die spezifische Monoacylierung und flexible Bindung an ein Polymerrückgrat bleiben die Eigenschaften der Saccharidreste auch in den Polymerisaten weitgehend erhalten.

Ein Gegenstand der Erfindung sind Verbindungen der Formeln I und Ia,

R-Y-CO-R₃-CO-O-A (I),

R-Y-CO-R₃-CO-O-CH₂-A₁ (Ia),

worin R eine radikalisch polymerisierbare Kohlenwasserstoffgruppe bedeutet, R₃ für eine direkte Bindung, lineares oder verzweigtes C₁-C₂₂-Alkylen, C₃-C₈-Cycloalkylen oder C₆-C₁₄-Arylen steht, A für den in einer 2- oder 3-Stellung um eine Hydroxylgruppe verminderten Rest eines cyclisch-oligomeren Kohlenhydrats oder eines solchen Kohlenhydratderivats steht, A₁ den um eine Hydroxymethylgruppe verminderten Rest eines monomeren oder linearen oder verzweigten oligomeren Kohlenhydrats oder eines solchen Kohlenhydratderivats bedeutet, und Y -O-, -NH- oder -N(C₁-C₆-Alkyl)- darstellt.

R enthält bevorzugt 2 bis 12, besonders bevorzugt 2 bis 10 und insbesondere bevorzugt 2 bis 8 C-Atome. Der Rest R kann Ethen- oder Ethingruppen als radikalisch polymerisierbare Gruppen enthalten. Bei R kann es sich zum Beispiel um Alkenyl, Alkinyl, Vinylphenyl oder Vinylbenzyl handeln. Einige Beispiele für Alkenyl sind Vinyl, Allyl, 1-Propen-2-yl, 1-Buten-2- oder -3- oder -4-yl, 2-Buten-3-yl, die Isomeren von Pentenyl, Hexenyl, Octenyl, Decenyl und Dodecenyl. Bevorzugt sind Vinyl, Allyl und 1-Propen-2-yl. Bei R als Alkinyl handelt es sich bevorzugt um einen Alkinylalkylrest, zum Beispiel HCC-CₘH₂ₘ-oder C₁-C₄-Alkyl-CC-CₘH₂ₘ-, worin m eine ganze Zahl von 1 bis 10, bevorzugt 1 bis 6 und besonders bevorzugt 1 bis 4 ist, und die Gesamtzahl der C-Atome 3 bis 12, bevorzugt 3 bis 8 und besonders bevorzugt 3 bis 6 beträgt. Einige Beispiele für Alkinyl sind Propargyl, 1-Butin-3- oder -4-yl, 1-Pentin-3- oder -4- oder-5-yl, 2-Pentin-4- oder -5-yl, 1-Hexin-3- oder -4- oder -5- oder -6-yl, 2-Hexin-4- oder -5- oder -6-yl, 3-Hexin-5- oder -6-yl.

Die Gruppe Y steht bevorzugt für -O-, -NH-, -NMethyl- oder -NEthyl- und besonders bevorzugt -O- oder -NH-.

R₃ enthält als Alkylen bevorzugt 2 bis 20 und besonders bevorzugt 4 bis 18 C-Atome. Einige Beispiele sind Methylen, 1,1-Ethyliden, 1,1- oder 2,2-Propyliden, 1,1- oder 2,2-Butyliden, 1,1-, 2,2- oder 3,3-Pentyliden oder -Hexyliden, 1,2-Ethylen, 1,2- oder 1,3-Propylen, 1,2-, 1,3-, 2,3- oder 1,4-Butylen, 1,2-, 1,3-, 1,4-, 1,5-, 2,3-, 2,4- oder 2,5-Pentylen, 1,2-, 1,3-, 1,4-, 1,5-, 1,6-, 2,3-, 2,4-, 2,5-, 2,6-Hexylen, die Isomeren von Heptylen, Octylen, Nonylen, Decylen, Undecylen, Dodecylen, Tridecylen, Tetradecylen, Pentadecylen, Hexadecylen, Heptadecylen, Octadecylen, Nonadecylen und Eicosylen.

R₃ enthält als Cycloalkylen bevorzugt 4 bis 6 und besonders bevorzugt 5 oder 6 C-Atome. Einige Beispiele sind Cyclopropylen, Cyclobutylen, Cyclopentylen, Cyclohexylen, Cycloheptylen und Cyclooctylen. R₃ bedeutet als Cycloalkylen bevorzugt 1,2- oder 1,3-Cyclopentylen und 1,2-, 1,3- und 1,4-Cyclohexylen.

R₃ bedeutet als Arylen bevorzugt C₆-C₁₄-Arylen. Beispiele sind 1,2-, 1,3- und 1,4-Phenylen, 2,3-, 2,7 oder 2,8-Naphthylen, und Biphenylene der Formel 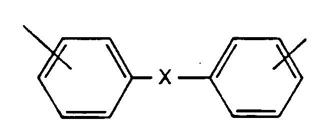 worin X eine direkte Bindung, -CH₂-, CH₃CH=, (CH₃)₂C=, Cyclohexyliden, -O-, -S-, -CO-, -CO₂-, -SO-, -SO₂-, -NH-, -CO-NH-, -N(C₁-C₄-Alkyl)- oder -CO-N(C₁-C₄-Alkyl)-bedeutet.

In einer bevorzugten Ausführungsform bedeutet R₃ lineares oder verzweigtes Alkylen mit 2 bis 20 C-Atomen, besonders bevorzugt 4 bis 14 C-Atomen.

Cyclische oligomere Kohlenhydrate, von denen sich der Rest A ableitet, sind bekannt Sie können zum Beispiel 6 bis 8 gleiche oder verschiedene Einheiten eines Monosaccharids enthalten. Beispiele für Monosaccharide sind nachfolgend erwähnt. Einige bevorzugte Beispiele sind α-, β- und γ-Cyclodextrin. Als Derivate kommen in 6-Stellung mit einem Monosaccharid, Oligosaccharid, C₁-C₁₂-Alkyl, C₂-C₄-Hydroxyalkyl oder C₁-C₁₂-Acyl substituierte Derivate in Frage, zum Beispiel 6-Methyl-, 6-Hydroxyethyl-, 6-Hydroxypropyl-, 6-Acetyl- und 6-Maltosylcyclodextrin.

Im Rahmen der vorliegenden Erfindung werden unter monomeren und linearen oder verzweigten cligomeren Kohlenhydraten Saccharide, beispielsweise Mono- und Oligosaccharide wie Mono-, Di-, Tri-, Tetra- und Pentasaccharide bis zu Decasacchariden verstanden. Die Oligosaccharide enthalten bevorzugt 2 bis 8 und besonders bevorzugt 2 bis 6 gleiche oder verschiedene Saccharideinheiten. In einer bevorzugten Ausführungsform handelt es sich bei den Mono- und Oligosacchariden um Aldosen oder Ketosen. In einer besonders bevorzugten Ausführungsform handelt es sich bei dem Monosaccharid um Aldopentosen, Aldohexosen, Ketopentosen oder Ketohexosen.

Beispiele für eine Aldopentose sind D-Ribose, D-Arabinose, D-Xylose oder D-Lyxose; für eine Aldohexose D-Allose, D-Altrose, D-Glucose, D-Mannose, D-Gulose, D-Idose, D-Galaktose oder D-Talose; für eine Ketopentose D-Ribulose oder D-Xylulose; für eine Ketohexose D-Psicose, D-Fructose, D-Sorbose oder D-Tagatose.

Beispiele für ein Disaccharid sind Trehalose, Maltose, Isomaltose, Cellobiose, Gentiobiose, Saccharose oder Lactose.

Als Trisaccharid sei beispielhaft Raffinose und Panose genannt.

Als weitere Oligomere kommen zum Beispiel oligomere Abbauprodukte von Polysacchariden in Frage, wie zum Beispiel von Stärke, Dextran, Cellulose, Curdlan, Pullulan und Chitin. Einige Beispiele sind Dextrine, Maltotetraose, Maltohexaose, Chitoheptaose und Sialyl-Lewis^{x}.

Als Derivate der monomeren und linearen oder verzweigten oligomeren Kohlenhydrate sind zum Beispiel solche zu nennen, die in 1- und/oder 2- und/oder 3-Stellung mit C₁-C₁₂-Alkyl, C₂-C₄-Hydroxyalkyl und C₁-C₁₂-Acyl substituiert sind. Weitere geeignete Derivate sind zum Beispiel natürliche und synthetische Nukleoside sowie Oligonukleotide aus 2 bis 20, bevorzugt 2 bis 10 gleiche oder verschiedene solcher Nukleoside.

In einer bevorzugten Ausführungsform stellt die Gruppe R Allyl, Propargyl, p-Vinylphenyl, p-Vinylbenzyl oder einen Rest der Formel R₁CH=CR₂- dar, worin R₁ H oder C₁-C₆-Alkyl bedeutet und R₂ H, C₁-C₆-Alkyl oder Phenyl darstellt.

Wenn R₁ Alkyl bedeutet, enthält es bevorzugt 1 bis 4 und besonders bevorzugt 1 oder 2 C-Atome. Bevorzugt ist das Alkyl linear. Einige Beispiele sind Methyl, Ethyl, n-Propyl und n-Butyl. Besonders bevorzugt stellt R₁ als Alkyl Methyl oder Ethyl dar.

In einer bevorzugten Ausführungsform stellt R₁ H, Methyl oder Ethyl dar. Besonders bevorzugt ist R₁ H.

Wenn R₂ Alkyl bedeutet, enthält es bevorzugt 1 bis 4 und besonders bevorzugt 1 oder 2 C-Atome. Bevorzugt ist das Alkyl linear. Einige Beispiele sind Methyl, Ethyl, n-Propyl n-Butyl, n- Pentyl und n-Hexyl. Bevorzugt ist R₂ H, Methyl, Ethyl, Propyl oder Butyl.

In einer besonders bevorzugten Ausführungsform bedeutet R₁ H und R₂ H, Methyl, Ethyl, n-Propyl oder n-Butyl. Ganz besonders bevorzugt stellen R₁ und R₂ je H dar.

Ein weiterer Gegenstand der Erfindung ist ein Verfahren zur Herstellung der Verbindungen der Formeln I und Ia, das dadurch gekennzeichnet ist, dass man einen Divinyldicarbonsäureester der Formel II

R₁CH=CR₂-O-CO-R₃-CO-O-R₂C=CHR₁ (II),

oder einem Monovinylester der Formel IIa

R"-Y-CO-R₃-CO-O-R₂C=CHR₁ (IIa),

worin R₁ und R₂ die nachfolgenden und R₃ und Y die zuvor angegebenen Bedeutungen haben, (a) mit einem cyclisch-oligomeren Kohlenhydrat der Formel A-OH oder dessen Derivaten umestert, worin A die zuvor angegebene Bedeutung hat, oder (b) in Gegenwart von lipolytischen Enzymen mit einem monomeren oder linearen oligomeren Kohlenhydrat der Formel A₁-CH₂OH oder deren Derivaten umestert, worin A₁ die zuvor angegebene Bedeutung hat, und (c) gegebenenfalls die erhaltenen Verbindungen der Formeln I und Ia, worin R eine radikalisch polymerisierbare Vinylestergruppe bedeutet, mit einem Alkohol der Formel R'-OH , einem Amin R'NH₂ oder einem Amin R'NHC₁-C₄-Alkyl umestert oder amidiert, wobei R' und R" unabhängig voneinander für eine nicht-vinylische radikalisch polymerisierbare Kohlenwasserstoffgruppe stehen.

Die Verbindungen der Formel II sind bekannt oder können nach bekannten Verfahren hergestellt werden, zum Beispiel nach den von D. Swern et al. in Organic Synthesis Coll. Vol. IV, Wiley New York, S. 977-980 (1963) oder von E. S. Rothman et al. im J. Org. Chem. 27, S. 3123-3127 (1962) beschriebenen Vorschriften. Die Kohlenhydrate der Formeln A-OH und A₁-CH₂OH sind ebenfalls bekannt und käuflich.

Die Umesterungen bei der Reaktionsstufe (a) werden zweckmässig in einem Puffer bei Temperaturen von 0 bis 150 °C durchgeführt. Dem Reaktionsgemisch werden vorteilhaft inerte, polare und mit Wasser mischbare Lösungsmittel zugegeben. Geeignete Lösungsmittel sind besonders Alkanole wie zum Beispiel Methanol, Ethanol, Propanol und Butanol oder Aceton, Tetrahydrofuran, Dioxan und Dimethylformamid. Die Isolierung der Reaktionsprodukte erfolgt in an sich bekannter Weise zum Beispiel durch Fällung, Filtration und anschliessende Trocknung. Die Produkte können durch Auswaschen, Umfällungen oder chromatograhische Verfahren gereinigt werden. Die Umesterungen bei der Reaktionsstufe (b) werden zweckmässig in einem inerten und polaren Lösungsmittel bei Temperaturen von 0 bis 150 °C durchgeführt. Geeignete Lösungsmittel sind besonders Diethylether, Dibutylether, t-Butyl-methylether, Tetrahydrofuran, Dioxan, Aceton, Methylisobutylketon, Hexan, Cyclohexan, Methylcyclohexan, Benzol, Toluol und Xylol. Die Isolierung und Reinigung der Produkte kann wie zuvor erfolgen. Umesterungen und Amidierungen gemäss der Verfahrensstufe (c) sind dem Fachmann geläufig. Als Beispiel für lipolytische Enzyme wird die Lipase von Humicola lanuginosa angegeben. Die Reaktionen werden zweckmässig unter Inertgas, zum Beispiel Edelgasen oder Stickstoff, durchgeführt.

Die Verbindungen der Formeln I und Ia sind einheitliche monoacylierte Monomere mit einer radikalisch polymerisierbaren Gruppe in der Estergruppe, die in bekannter Weise zum Beispiel unter Zusatz von Radikalinitiatoren zu linearen Homopolymeren oder linearen beziehungsweise vernetzten Copolymeren polymerisiert werden können, bei denen die hydrophile Kohlenhydratgruppe über eine flexible Seitenkette an das Polymerrückgrat gebunden ist und die Eigenschaften dieser Gruppe weitgehend erhalten sind.

Ein weiterer Gegenstand der Erfindung sind Polymere, die bezogen auf das Polymer
i) 0,1 bis 100 Mol-% mindestens eines Strukturelements der Formeln III und/oder IIIa enthalten, 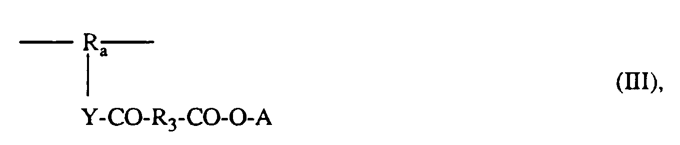 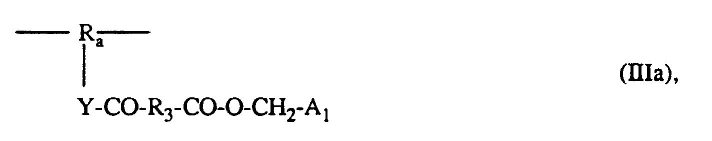 worin Rₐ den Rest einer radikalisch polymerisierten Gruppe darstellt und R₃, A, A₁ und Y die zuvor angegebenen Bedeutungen haben,
ii) 99,9 bis 0 Mol-% mindestens eines von den Formeln III und IIIa verschiedenen Strukturelements eines radikalisch polymerisierten Olefins, und
iii) 80 bis 0 Mol-% mindestens eines Strukturelements eines radikalisch polymerisierten Diolefins, wobei sich die Molprozente zu 100 % addieren.

Die erfindungsgemässen Polymeren können ein mittleres Molekulargewicht (Gewichtsmittel) von 500 bis 2000000, bevorzugt 1000 bis 1000000 und besonders bevorzugt 1000 bis 500000 aufweisen. Die erfindungsgemässen linearen Polymere können zum Beispiel Molekulargewichte von 500 bis 200000, bevorzugt 500 bis 100000 und besonders bevorzugt 500 bis 50000 aufweisen.

Rₐ enthält bevorzugt 2 bis 12, besonders bevorzugt 2 bis 10 und insbesondere bevorzugt 2 bis 8 C-Atome. Bei dem Rest Rₐ kann es sich um dreiwertige Ethantriyl-, Phenylen-ethylen-, Phenylenmethyl-ethylen- oder Ethentriyl-gruppen handeln. Die Ethantriylgruppen können unsubstituiert oder mit Alkyl substituiert sein, wobei die so substituierten Ethantriylgruppen bevorzugt 2 bis 12, besonders bevorzugt 2 bis 10 und insbesondere bevorzugt 2 bis 8 C-Atome enthalten. Einige Beispiele sind Ethantriyl, Propan-1,2,3-triyl, Butan- 1,2,4-triyl, Pentan-1,2,5-triyl, Hexan-1,2,6-triyl, Heptan-1,2,7-triyl, Octan-1,2,8-triyl, -CH₂-CH(C₆H₄-)- und -CH₂-CH(C₆H₄CH₂-)-. Bei der Ethentriylgruppe kann es sich zum Beispiel um solche der Formeln -HC=C(CₘH₂ₘ-)- oder -(C₁-C₉-Alkyl)C=C(CₘH₂ₘ-)- handeln, worin m eine ganze Zahl von 1 bis 10, bevorzugt 1 bis 6 und besonders bevorzugt 1 bis 4 ist, und die Gesamtzahl der C-Atome 3 bis 12, bevorzugt 3 bis 8 und besonders bevorzugt 3 bis 6 beträgt Ein Beispiel ist -HC=C(CH₂-)-.

Für R₃, A, A₁ und Y gelten die zuvor aufgeführten Ausgestaltungen und Bevorzugungen.

Die Komponente i) kann zum Beispiel in einer Menge von 0,5 bis 100 Mol-%, bevorzugt 1 bis 100 Mol-%, bevorzugter 5 bis 100 Mol-%, besonders bevorzugt 10 bis 100 Mol-%, und insbesondere bevorzugt 20 bis 100 Mol-% enthalten sein. Der Gehalt an den Comonomereinheiten ii) und iii) richtet sich im wesentlichen nach den gewünschten Eigenschaften. Die Komponente ii) kann zum Beispiel in einer Menge von 99,5 bis 0,5 Mol-%, bevorzugt 99 bis 1 Mol-%, bevorzugter 95 bis 1 Mol-%, besonders bevorzugt 90 bis 1 Mol-%, und insbesondere bevorzugt 80 bis 1 Mol-% enthalten sein. Die Komponente iii) kann zum Beispiel in einer Menge von 70 bis 0,01 Mol-%, bevorzugt 60 bis 0,05 Mol-%, bevorzugter 50 bis 0,1 Mol-%, besonders bevorzugt 40 bis 0,5 Mol-%, und insbesondere bevorzugt 30 bis 1 Mol-% enthalten sein.

In einer bevorzugten Ausführungsform stellt die Gruppe Rₐ Reste der Formeln -CH₂-CH(CH₂-)-, -CH₂-CH(C₆H₄-)-, -CH₂-CH(C₆H₄CH₂-)-, -HC=C(CH₂-)- oder -(R₁ )CH-C(R₂)= dar, worin R₁ H oder C₁-C₆-Alkyl bedeutet und R₂ H, C₁-C₆-Alkyl oder Phenyl darstellt.

In einer besonders bevorzugten Ausführungsform entsprechen die Strukturelemente der Komponente i) den Formeln IV oder IVa, 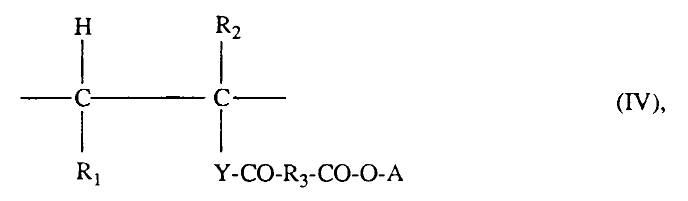 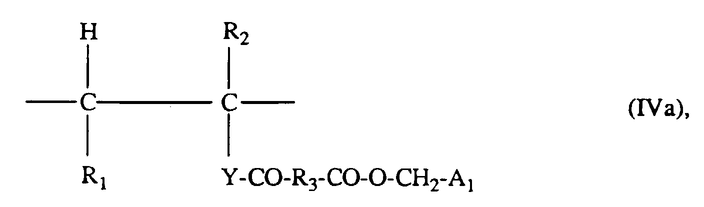 worin R₁ H oder C₁-C₆-Alkyl bedeutet und R₂ H, C₁-C₆-Alkyl oder Phenyl darstellt, und A, A₁, R₃ und Y die zuvor angegebenen Bedeutungen haben, einschliesslich bevorzugter Ausführungsformen.

Wenn R₁ Alkyl bedeutet, enthält es bevorzugt 1 bis 4 und besonders bevorzugt 1 oder 2 C-Atome. Bevorzugt ist das Alkyl linear. Einige Beispiele sind Methyl, Ethyl, n-Propyl und n-Butyl. Besonders bevorzugt stellt R₁ als Alkyl Methyl oder Ethyl dar.

In einer bevorzugten Ausführungsform stellt R₁ H, Methyl oder Ethyl dar. Besonders bevorzugt ist R₁ H.

Wenn R₂ Alkyl bedeutet, enthält es bevorzugt 1 bis 4 und besonders bevorzugt 1 oder 2 C-Atome. Bevorzugt ist das Alkyl linear. Einige Beispiele sind Methyl, Ethyl, n-Propyl n-Butyl, n- Pentyl und n-Hexyl. Bevorzugt ist R₂ H, Methyl, Ethyl, Propyl oder Butyl.

In einer besonders bevorzugten Ausführungsform bedeutet R₁ H und R₂ H, Methyl, Ethyl, n-Propyl oder n-Butyl. Ganz besonders bevorzugt stellen R₁ und R₂ je H dar.

Olefinmonomere, von denen sich die Strukturelemente der Komponente ii) ableiten können, sind in grosser Vielzahl bekannt. Bevorzugt handelt es sich um Ethylen, das unsubstituiert oder mit Halogen, -OH, -CN, Pyrrolidonyl, C₁-C₁₂-Alkyl, Phenyl, C₁-C₄-Alkylphenyl, C₁-C₄-Alkoxyphenyl, Halogenphenyl, Hydroxyphenyl, C₁-C₄-Alkyl-hydroxy-phenyl, C₁-C₄-Alkoxy-hydroxy-phenyl, Chlor- oder Brom-hydroxy-phenyl, C₁-C₄-Alkoxy, Phenoxy, C₁-C₄-Alkylphenoxy, Benzyl, Benzyloxy, -COO^{⊖}M^{⊕}, -COOR₄, -COOCH₂CH(OH)CH₂OH, -COBR₅-OH, -COO-(R₆R₇SiO)ₙ-SiR₆R₇R₈, -COBR₅-O-(R₆R₇SiO)ₙ-SiR₆R₇R₈, -CONH₂, -CONH(C₁-C₆-Alkyl), -CON(C₁-C₆-Alkyl)₂ oder -OCO-R₄ substituiert ist, worin M^{⊕} für H^{⊕}, ein Alkalimetallkation oder ein Ammoniumkation steht, R₄ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₅ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, B für -O-, -N(C₁-C₆-Alkyl)- oder -NH- steht, R₆, R₇ und R₈ unabhängig voneinander C₁-C₆-Alkyl oder C₁-C₆-Alkoxy bedeuten und n für eine Zahl von 1 bis 30 steht.

R₄ kann lineares oder verzweigtes C₁-C₁₈-, bevorzugt C₁-C₁₂- und besonders C₁-C₆-Alkyl sein. R₄ stellt als Cycloalkyl besonders Cyclopentyl oder Cyclohexyl dar. Bei R₄ als (C₁-C₁₂-Alkyl)-cycloalkyl ist das Cycloalkyl besonders Cyclopentyl oder Cyclohexyl und die Alkylgruppe kann linear oder verzweigt sein und enthält bevorzugt 1 bis 6, besonders 1 bis 4 C-Atome. Bedeutet R₄ Alkylphenyl oder Alkylbenzyl, so kann die Alkylgruppe linear oder verzweigt sein und enthält bevorzugt 1 bis 6, besonders 1 bis 4 C-Atome.

R₅ enthält als Alkylen bevorzugt 2 bis 12, besonders bevorzugt 2 bis 8, und insbesondere bevorzugt 2 bis 6 C-Atome. Beispiele sind Ethylen und die Isomeren von Propylen, Butylen, Pentylen, Hexylen, Heptylen, Octylen, Nonylen, Decylen, Undecylen, Dodecylen, Tetradecylen, Hexadecylen und Octadecylen. Bevorzugt sind Ethylen, 1,2- und 1,3-Propylen, 1,2-, 1,3- und 1,4-Butylen, 1,2-, 1,3-, 1,4- und 1,5-Pentylen und 1,2-, 1,3-, 1,4-, 1,5- und 1,6-Hexylen.

R₅ in der Bedeutung von Poly(oxaalkylen) enthält bevorzugt 2 bis 4 Oxaalkyleneinheiten, und bevorzugt 2 bis 4, besonders bevorzugt 2 oder 3 C-Atome im Alkylenrest.

R₅ in der Bedeutung von Cycloalkylen stellt insbesondere Cyclopentylen oder Cyclohexylen dar.

R₆, R₇ und R₈ sind bevorzugt C₁-C₄-Alkyl oder C₁-C₄-Alkoxy und insbesondere bevorzugt Methyl, Ethyl, Methoxy oder Ethoxy. Bei n handelt es sich bevorzugt um Zahlen von 1 bis 20, besonders bevorzugt von 1 bis 10.

Bei M^{⊕} in der Bedeutung eines Ammoniumkations kann es sich zum Beispiel um NH₄^{⊕} oder die Kationen eines primären Amins mit 1 bis 12 C-Atomen, eines sekundären Amins mit 2 bis 18 C-Atomen, eines tertiären Amins mit 3 bis 24 C-Atomen, oder um quaternäres Ammonium mit 4 bis 30, bevorzugt 4 bis 20 C-Atomen handeln.

In einer bevorzugten Ausführungsform handelt es sich bei Komponente ii) um Struktureinheiten der Formel IV 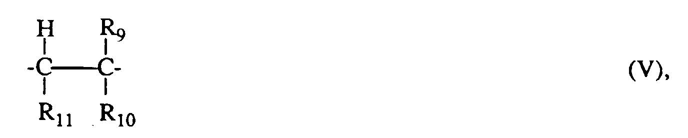 worin R₁₁ für H, C₁-C₆-Alkyl -COOR₄ oder -COO^{^{⊖}}M^{⊕} steht, R₉ H, F, Cl, CN oder C₁-C₆-Alkyl bedeutet, R₁₀ H, -F, -Cl, -CN, Pyrrolidonyl, R₁₂O-, C₁-C₁₂-Alkyl, -OH, -COO^{⊖}M^{⊕} , -COOR₄, -COOCH₂CH(OH)CH₂OH, -CONH₂, -CONH(C₁-C₄-Alkyl), -CON(C₁-C₄-Alkyl)₂, -COBR₅-OH, -OCO-R₄, -COO-(R₆R₇SiO)ₙ-SiR₆R₇R₈, -COBR₅-O-(R₆R₇SiO)ₙ-SiR₆R₇R₈, Phenyl oder mit -OH und/oder ein oder zwei Methyl, Methoxy, Cl oder Br substituiertes Phenyl darstellt, M^{⊕} für H^{⊕}, ein Alkalimetallkation oder ein Ammoniumkation steht, R₄ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₁₂ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, B für -O-, -N(C₁-C₄-Alkyl)- oder -NH- steht, und R₆, R₇ und R₈ Methyl, Ethyl, Methoxy oder Ethoxy bedeuten, und n für 0 oder eine Zahl von 1 bis 20, bevorzugt 1 bis 12 bedeutet.

R₁₁ steht bevorzugt für H. Bedeutet R₁₁ Alkyl, so handelt es sich bevorzugt um Methyl oder Ethyl. Bedeutet R₁₁ -COOR₄, so stellt R₄ bevorzugt C₁-C₁₂-, besonders C₁-C₆-Alkyl dar.

Bedeutet R₉ Alkyl, so handelt es sich bevorzugt um C₁-C₄-Alkyl, z.B. Methyl, Ethyl, n-Propyl und n-Butyl. R₉ steht bevorzugt für H, Cl oder C₁-C₄-Alkyl.

Bedeutet R₁₀ die Gruppe R₁₂-O-, so stellt R₁₂ bevorzugt C₁-C₁₂-, besonders C₁-C₆-Alkyl dar. Bedeutet R₁₀ Alkyl, so enthält es bevorzugt 1 bis 6, besonders 1 bis 4 C-Atome. Bedeutet R₁₀ die Gruppe -COOR₄, so stellt R₄ bevorzugt C₁-C₁₂-, besonders C₁-C₆-Alkyl, Cyclopentyl oder Cyclohexyl dar. Bedeutet R₁₀ die Gruppe -OCO-R₄, so stellt R₄ bevorzugt C₁-C₁₂-, besonders C₁-C₆-Alkyl, Phenyl oder Benzyl dar.

Wenn R₁₀ die Gruppe -COOR₅OH darstellt, so gelten die zuvor für R₅ angegebenen Bevorzugungen.

Bedeutet R₁₀ die Gruppen -CONH(C₁-C₄-Alkyl) oder -CON(C₁-C₄-Alkyl)₂, so handelt es sich bevorzugt um -CONHCH₃, -CONHC₂H₅, CON(CH₃)₂ oder -CON(C₂H₅)₂.

In einer bevorzugten Ausführungsform stehen R₁₁ für H, R₉ für H, -F, -Cl, Methyl oder Ethyl, und R₁₀ für Pyrrolidonyl, -F, -Cl, -CN, -OH, C₁-C₄-Alkyl, C₁-C₆-Alkoxy, -COO-C₁-C₆-Alkyl, -COO-R₅-OH, -COOM^{⊕}, -OOC-C₁-C₆-Alkyl, -COOCH₂CH(OH)CH₂OH, -CONH₂, -CONH(C₁-C₄-Alkyl), -CON(C₁-C₄-Alkyl)₂, -COO-(R₆R₇SiO)ₙ-SiR₆R₇R₈, -COBR₅-O-(R₆R₇SiO)ₙ-SiR₆R₇R₈, Phenyl, Methylphenyl, Dimethylphenyl, Chlorphenyl, Dichlorphenyl, Dibromphenyl, Methoxyphenyl, Dimethoxyphenyl, Hydroxyphenyl, worin M^{⊕} Trialkylammonium mit 1 bis 4 C-Atomen in den Alkylgruppen bedeutet, und R₅ C₂-C₆-Alkylen darstellt, und R₆, R₇ und R₈ Methyl, Ethyl, Methoxy oder Ethoxy bedeuten, und n für 0 oder eine Zahl von 1 bis 12 steht.

In einer ganz besonders bevorzugten Ausführungsform stehen in Formel V R₁₁ für H, R₉ für H oder Methyl und R₁₀ für Pyrridonyl, -CN, -COOH, -COO-C_{y}H_{2y}-OH mit y gleich 2 bis 6, besonders 2 oder 3, -CON(CH₃)₂, -COO-CH₂CH(OH)CH₂OH und -COO-CH₂CH₂-O-[Si(OCH₃)₂-O]ₙ-Si(OCH₃)₃ oder -COO-CH₂CH₂-O-[Si(OC₂H₅)₂-O]ₙ-Si(OC₂H₅)₃ mit n gleich 1 bis 8 und bevorzugt 2 bis 6.

Bei den erfindungsgemässen Copolymeren kann es sich um Blockpolymere oder um Copolymere mit einer alternierenden oder statistischen Verteilung der Struktureinheiten handeln.

Bei der Komponente iii) kann es sich zum Beispiel um Struktureinheiten von Butadien, Isopren und Chloropren handeln. Weitere geeignete Struktureinheiten können sich von Diacrylaten oder Dimethacrylaten von Diolen oder von Diacryl- oder Dimethacrylamiden von Diaminen ableiten. Bei den Alkoholen und Diaminen kann es sich um solche der Formel HY-CₓH₂ₓ-YH handeln, worin x für eine Zahl von 2 bis 12, bevorzugt 2 bis 6 steht, und Y -O-, -NH- oder -N(C₁-C₄-Alkyl)- bedeutet. Als Diole kommen auch Polyoxaalkylendiole der Formel HO-(CₙH₂ₙO)_{y}-H in Frage, worin n für eine Zahl von 2 bis 6, bevorzugt 2 bis 4 und besonders bevorzugt 2 oder 3 steht, und y für eine Zahl von 2 bis 20, bevorzugt 2 bis 10, besonders bevorzugt 2 bis 6 und insbesondere bevorzugt 2 bis 4 steht. Einige Beispiele sind Ethylenglykol, 1,2- und 1,3-Propylenglykol, 1,2-, 1,3- und 1,4-Propylenglykol und die Isomeren von Pentylenglykol, Hexylenglykol, Heptylen-glykol, Octylenglykol, Nonylenglykol, Decylenglykol, Undecylenglykol und Dodecylenglykol, Diethylenglykol, Triethylenglykol, Tetraethylenglykol, Di-1,2-Propylenglykol, Tri-1,2-propylenglykol, oligomere Polyaxaalkylenglykole mit 4 bis 12 gleichen oder verschiedenen Oxaethylen oder Oxapropylenresten.

Die erfindungsgemässen Polymeren können als vierte Komponente iiii) bis zu 10 Mol-%, bevorzugt 0,01 bis 5 Mol-% Reste von trifunktionellen ethylenisch ungesättigten Verbindungen enthalten, wobei die Molprozente im Polymer 100 Mol-% betragen. Als Beispiele seien die Acrylat- und Methacrylattriester von Trimethylolpropan oder Methyltrimethylolpropan genannt.

Die Herstellung der erfindungsgemässen Polymeren kann nach üblichen Methoden durch radikalische einschliesslich photochemisch-radikalische Polymerisation erfolgen, wobei als Methoden zum Beispiel Block-, Emulsions-, Lösungs- oder Grenzflächenpolymerisation in Frage kommen. Auch die Isolierung und Reinigung kann nach den in der Polymerchemie üblichen Verfahren vorgenommen werden. Weitere Einzelheiten sind in den Beispielen beschrieben.

Die erfindungsgemässen Polymeren sind farblose transparente bis weisse und opake Feststoffe, die eine sehr hohe Hydrophilie aufweisen. Je nach Zusammensetzung sind sie wasserlöslich oder in Wasser oder anderen dipolaren und aprotischen oder protischen Lösungsmitteln löslich oder quellbar, und können Gele oder Hydrogele oder nichtquellbare Polymere mit stark hydrophilen Oberflächen bilden. Die Polymeren können je nach Zusammensetzung hydrophil, amphiphil oder hydrophob sein. Die Eigenschaften der Polymeren können durch die Auswahl und Mengen von Monomeren und des Spacers in den erfindungsgemässen Monomeren spezifisch eingestellt werden. Die Monomeren und Polymeren sind einfach zugänglich. Die Polymeren sind teilweise physiologisch unbedenklich und zeichnen sich durch ihre gleichbleibenden guten biologischen und physikochemischen Eigenschaften aus. Die erfindungsgemässen Polymeren sind auch teilweise biologisch abbaubar. Diese Polymeren können breite Anwendungen in der Technik finden.

Die erfindungsgemässen Polymere haben filmbildende Eigenschaften. Beim Eindampfen von wässrigen oder organischen Lösungen bilden sich transparente, feste und gegebenenfalls Wasser enthaltende Filme, die luft- und feuchtigkeitsdurchlässig sind. Auf Grund dieser Eigenschaft und ihrer wasserspeichernden Wirkung eignen sie sich auch als Befeuchtigungsmittel für die Haut oder die Schleimhäute in kosmetischen und pharmazeutischen Präparaten, als Mittel zur Aufrechterhaltung der Gelenkbeweglichkeit und für Wundverbände. Kosmetische Präparate sind zum Beispiel Haut- und Haarpflegemittel und Deodorantien. Die erfindungsgemässen Polymeren, besonders daraus hergestellte Gele, eignen sich ferner zur Herstellung von Präparaten mit einer über einen längeren Zeitraum kontrollierten Wirkstoffabgabe, zum Beispiel pharmazeutischen und pestiziden Wirkstoffen oder Aromastoffen.

Die erfindungsgemässen wasserlöslichen Polymere weisen in wässrigen Lösungen auch eine viskositätssteigernde und dispergierende Wirkung auf, und können als Tenside und Verdickungsmittel verwendet werden. Sie eignen sich zum Beispiel als als Zusätze in Suspensionen, Emulsionen, Dispersionen und wässrigen Lösungen, zum Beispiel bei der Herstellung von Lebensmitteln oder biologisch aktiven Wirkstoffkonzentraten sowie Farbstoff- und Pigmentzubereitungen.

Aus den erfindungsgemässen Polymeren kann man in an sich bekannter Weise Filme und Folien herstellen, die als Membranen oder Wundverbände verwendbar sind, oder man kann in an sich bekannter Weise Kapseln für Wirkstoffe oder umhüllte Wirkstoffe herstellen, wobei die Wirkstoffabgabe an die Umgebung verzögert und kontinuierlich erfolgt.

Man kann auch feste Trägermaterialien wie zum Beispiel Metalle, Halbmetalle, Keramiken, Gläser, Metall- und Halbmetalloxide oder -nitride, Holz, Papier und Kunststoffe mit den erfindungsgemässen Polymeren beschichten und diese beschichteten Materialien zum Beispiel als Basismaterial für Sensoren verwenden.

Die erfindungsgemässen Polymere können zum Beispiel auch für die Herstellung von Weichkontaktlinsen aus entsprechenden Hydrogelen oder von Hartkontaktlinsen mit permanent hydrophilen Oberflächen oder zur Modifizierung der Oberflächen von Kontaktlinsen oder zur Reinigung von Kontaktlinsen verwendet werden.

Ein weiterer Gegenstand der Erfindung ist die Verwendung der wasserlöslichen erfindungsgemässen Polymeren als Verdickungsmittel und Tenside.

Ein anderer Gegenstand der Erfindung ist die Verwendung der erfindungsgemässen Polymeren als Trägermaterial für Wirkstoffe für eine kontrollierte Wirkstoffabgabe.

Ein weiterer Gegenstand der Erfindung ist die Verwendung der erfindungsgemässen Polymeren zur Herstellung, Modifizierung oder Reinigung von Kontaktlinsen.

Die nachfolgenden Beispiele erläutern die Erfindung näher.

### A) Herstellung von Ausgangsprodukten

### Beispiel A1: α-, ω-Dicarbonsäuredivinylester

Die Ester werden gemäss der Vorschrift von Swern, D., Jordan, E.F., in Organic Syntheses Coll. Vol. IV, Wiley, New York, 1963, S. 977-980 hergestellt.

2 mol Dicarbonsäure werden unter Stickstoff in einem sieben- bis achtfachen molaren Ueberschuss von Vinylacetat suspendiert und mit 1 g Li(PdCl₂) versetzt. Die Reaktion wird durch Erhitzen und die Zugabe von wenigen Tropfen konzentrierter Schwefelsäure gestartet. Das Gemisch wird 20 h unter Rückfluss gerührt. Anschliessend werden 1 g Natriumacetat und 10 g Aktivkohle zugegeben, die Lösung filtriert und eingeengt. Der Rückstand wird mit 400 ml Diethylether verdünnt, fünfmal mit jeweils 200 ml einer kalt gesättigten Natriumhydrogencarbonatlösung extrahiert und mit Wasser gewaschen. Die getrocknete Etherphase wird filtriert und eingeengt. Zur Reinigung wird das Gemisch mit Dichlormethan über eine Kieselgelsäule getrennt. Die durchschnittlichen Ausbeuten betragen 50 bis 90 %.

Auf diese Art hergestellt wurden: 1,4-Divinylbutandioat, 1,6-Divinylhexandioat, 1,12-Divinyldodecandioat, 1,16-Divinylhexadecandioat, 1,22-Divinyldocosandioat, 4,4'-Divinylbiphenyldioat.

α-, ω-Dicarbonsäurediisopropenylester werden gemäss der Vorschrift von Rothman, E.S., Serota, S., Perlstein, T., Swern, D., J. Org. Chem. 27(1962) 3123-3127 hergestellt.

### B) Herstellung von Monomeren

### Beispiel B1: γ-Cyclodextrinyl-vinyl-hexadecandioat

2,5 g γ-Cyclodextrin (1,92 mmol) werden in 60 ml Phosphatpuffer (0,1 mol/l, pH 7.0) und 140 ml Ethanol gelöst und mit 5,0 g 1,16-Divinylhexadecandioat (17,5 mmol) versetzt. Das Gemisch wird 68 h bei 50°C gerührt. Anschliessend wird die Lösung eingeengt und mit Wasser auf 300 ml aufgefüllt. Mit je 200 ml Diethylether wird zweimal extrahiert und die wässrige Phase lyophilisiert Das Rohprodukt wird in 25 ml Wasser suspendiert und mit 150 ml Aceton versetzt. Nach Filtration und Lyophilisation werden 0,68 g (23 %) Produkt erhalten.

### Beispiel B2: β-Cyclodextrinyl-vinyl-dodecandioat

5 g β-Cyclodextrin (4,4 mmol) werden in 120 ml Phosphatpuffer (0,1 mol/l, pH 7.0) und 280 ml Ethanol suspendiert und mit 10,0 g Divinyldodecandioat (43,4 mmol) versetzt. Das Gemisch wird 30 h bei 50°C gerührt. Anschliessend wird die Lösung eingeengt und mit Wasser auf ein Volumen von 200 ml gebracht. Die Suspension wird zweimal mit je 200 ml Diethylether gewaschen und am Lyophilisator getrocknet. Das Rohgemisch wird in 50 ml Wasser suspendiert und es werden 270 ml Aceton zugegeben. Das Filtrat liefert nach Lyophilisation 2,4 g Produkt (40 %).

### Beispiel B3: α-Cyclodextrinyl-vinyl-hexandioat

50 g α-Cyclodextrin (51 mmol) werden in 3,5 1 Phosphatpuffer (0,1 mol/l, pH 7.0) und 1,5 1 Ethanol gelöst und mit 50 g Divinylhexandioat (0,25 mol) versetzt. Das Gemisch wird 30 h bei 50°C gerührt. Anschliessend wird die Lösung auf 1 l eingeengt, filtriert und dreimal mit je 1 1 Diethylether extrahiert. Die wässrige Phase wird lyophilisiert und das Rohprodukt in 940 ml Wasser gelöst und mit 5,64 1 Aceton umgefällt. Nach Filtration und dem Eindampfen bis zur Trockene verbleiben 28,35 g, welche in 300 ml Wasser gelöst und mit schwach basischem Anionenaustauscher (Dowex IRA-93) gerührt werden. Die Lyophilisation des Filtrats ergibt 17,4 g Produkt (30 %).

¹H-NMR (250 MHz, D₂O): 1,68 ppm (s, breit, 2(-βCH₂-)), 2,43 ppm (s, breit, 2(-αCH₂)), 3,33.bis 3,95 ppm (Cyclodextrin), 4,03 ppm (m, H-3A), 4,92 ppm (s, 5 H-1), 5,11 ppm (d, H-1A), 5,24 ppm (m, H-2A), 7,08 ppm (dt, -HC=CH₂).

### Beispiel B4: β-Cyclodextrinyl-vinyl-hexandioat

10 g β-Cyclodextrin (7,7 mmol) und 10 g Divinylhexandioat (50,5 mmol) werden in 700 ml Phosphatpuffer (0,1 mol/l, pH 7.0) und 300 ml Ethanol suspendiert. Die auf 50°C erwärmte Lösung wird 20 h gerührt und anschliessend auf 0,5 1 eingeengt. Nach dreimaligem Waschen mit je 250 ml Diethylether wird die wässrige Phase filtriert und lyophilisiert. Das Rohgemisch wird in 180 ml Wasser gelöst und mit 1080 ml Aceton versetzt. Nach Filtration wird die Lösung eingeengt und lyophilisiert. Es werden 6,74 g Produkt (61 %) erhalten.

### Beispiel B5: α-Cyclodextrinyl-vinyl-butandioat

1,34 g α-Cyclodextrin (1,37 mmol) werden in 75 ml Phosphatpuffer (69 mmol/l, pH 7.0) und 32 ml Ethanol gelöst. Nach Zusatz von 2,68 g Divinylbutandioat (15,75 mmol) wird 40 h bei 50°C gerührt. Anschliessend wird auf die Hälfte des Volumens eingeengt und mit Wasser ein Volumen von 200 ml eingestellt. Die Lösung wird zweimal mit je 200 ml Diethylether gewaschen und lyophilisiert. Das Rohprodukt wird in 32 ml Wasser aufgenommen und mit 192 ml Aceton versetzt. Nach Filtration und Lyophilisation wird die Substanz in 68 ml Wasser gelöst und mit schwach basischem Anionenaustauscher (Amberlite IRA-93) gerührt. Erneute Filtration und Gefriertrocknung liefern 300 mg (20 %) des Produktes.

### Beispiel B6: Maltosylcyclodextrinyl-vinyl-hexandioat

5 g Maltosylcyclodextrin (3,9 mmol) werden in 350 ml Phosphatpuffer (69 mmol/l, pH 7.0) und 150 ml Ethanol gelöst und mit 5 g (25 mmol) Divinylhexandioat versetzt. Nach 20 h Rühren bei 50°C wird das Gemisch abgekühlt und zweimal mit je 250 ml Diethylether extrahiert. Die wässrige Phase wird lyophilisiert und in 91 ml Wasser aufgenommen. Das bei Zugabe von 546 ml Aceton ausgefallene Material wird abfiltriert und das Filtrat eingeengt Lnd getrocknet. Es werden 2,2 g Produkt (39 %) erhalten.

### Beispiele B7-B13: 6-O-Glucosyl-vinyl-decandioat

0,5 g D-Glucose (2,78 mmol), 2,0 g Divinyldecandioat (7,87 mmol) und 0,5 g Lipase (Humicola lanuginosa) werden eingewogen und in 50 ml Aceton suspendiert. Das Gemisch wird mehrere Tage bei 50°C gerührt, dann verdünnt und filtriert. Das Filtrat wird mit Ethylacetat versetzt und der Niederschlag abgetrennt. Die Lösungsmittel werden abgedampft und der Rückstand in Ethylacetat aufgenommen und dann Hexan zugegeben. Der Ueberstand wird digeriert. Das Produkt wird als leicht gelbes Oel mit 0,924 g (85 %) erhalten. Die Reinigung durch Kieselgelsäulenchromatographie mit Dichlormethan/Methanol (5:1) ergibt 48 % Produkt).
1H-NMR (250 MHz, deuteriertes Aceton): 1,20 ppm (s, 8 H, γ,γ',δ,δ'-CH₂-), 1,48 ppm (m, 4 H, β,β'-CH₂-), 2,19 ppm (t, 2 H, α-CH₂-), 2,31 ppm (t, 2 H, α'-CH₂-), 3,19 ppm (m, 2 H, H-4, H-2), 3,57 ppm (t, 1 H, H-3), 3,84 ppm (m, 1 H, H-5), 4,03 ppm (q, 1 H, H-6a), 4,20 ppm (d, 1 H, H-6b), 4,47 ppm (d, 1H, -CH=CH₂a), 4,72 ppm, (d, 1 H, -CH=CH₂b), 4,98 ppm (d, 1 H, H-1), 7,17 ppm (dd, 1 H, -CH=CH₂).

Die in der nachfolgenden Tabelle aufgeführten Produkte werden analog hergestellt.

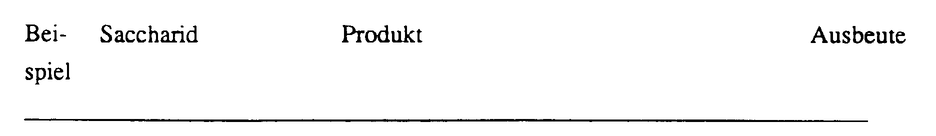

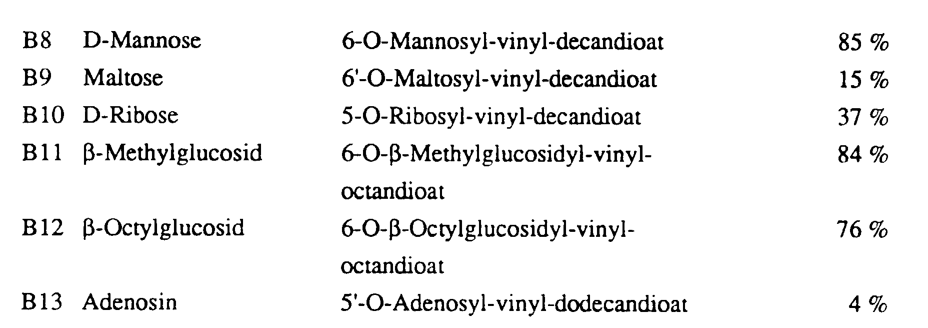

### C) Herstellung von Polymeren

### 3.a) Latexpolymerisate

Gemäss den Patentbeispielen von: Noda, I., US Patent 4,735,843, 5. April 1988. In dem Patent werden als Emulgatoren für Latexpolymerisationen Oligooleylethoxylat ("Volpo 20", Beispiele 1, 2, 4, 6) und Butadien-Ethylen-Blockcopolymer (Beispiel 3) beschrieben. Diese werden hier durch monofunktionalisierte Cyclodextrine mit emulgierenden Eigenschaften ersetzt (β-Cyclodextrinyl, vinyldodecandioat und γ-Cyclodextrinyl, vinylhexadecandioat).

Beispiel C1: β-Cyclodextrinyl-vinyl-dodecandioat-Isopren-Styrol-Latex 120 mg β-Cyclodextrinyl-vinyl-dodecandioat und 60 mg Kaliumperoxodisulfat werden unter Argon in einen Kolben gewogen und in 27 ml mit Argon gespültem, bidestilliertem Wasser gelöst. Dazu werden 50 µl Dodecylmercaptan, 0,77 ml Styrol und 3,08 ml Isopren gegeben. Der Kolben wird verschlossen und unter Rühren wird die Reaktion durch Erwärmen auf 50°C gestartet. Nach 16 h wird der Latex als weissliche Emulsion erhalten.

Ein transparenter, wasserbeständiger und hydrophiler Film (Kontaktwinkel ca. 45°) wird erhalten, wenn der gemäss Beispiel C1 beschriebene Latex auf einer Glasfläche ausgestrichen und 3 Tage unter einem Heissluftstrom von 100°C getrocknet wird.

### Beispiel C2-C13:

12 mg Azo-bis-isobutyronitril und 200 mg (0,18 mmol) α-Cyclodextrinyl-vinyladipat werden unter Argon in einem 5 ml-Kolben eingewogen und in 1 ml argonisiertem Lösungsmittel gelöst. Das Comonomer (0,36 mmol) wird zugetropft, der Kolben verschlossen und 18 bis 36 h bei 60°C gerührt. Das Produkt wird nach dem Abkühlen mit Essigester gefällt und abfiltriert. Eine 10%ige wässrige Lösung des Rückstands wird mit dem Fünffachen des Volumens mit Aceton versetzt und der Niederschlag abfiltriert und getrocknet, die Ausbeute wird bestimmt

Die Ergebnisse befinden sich in der nachfolgenden Tabelle:

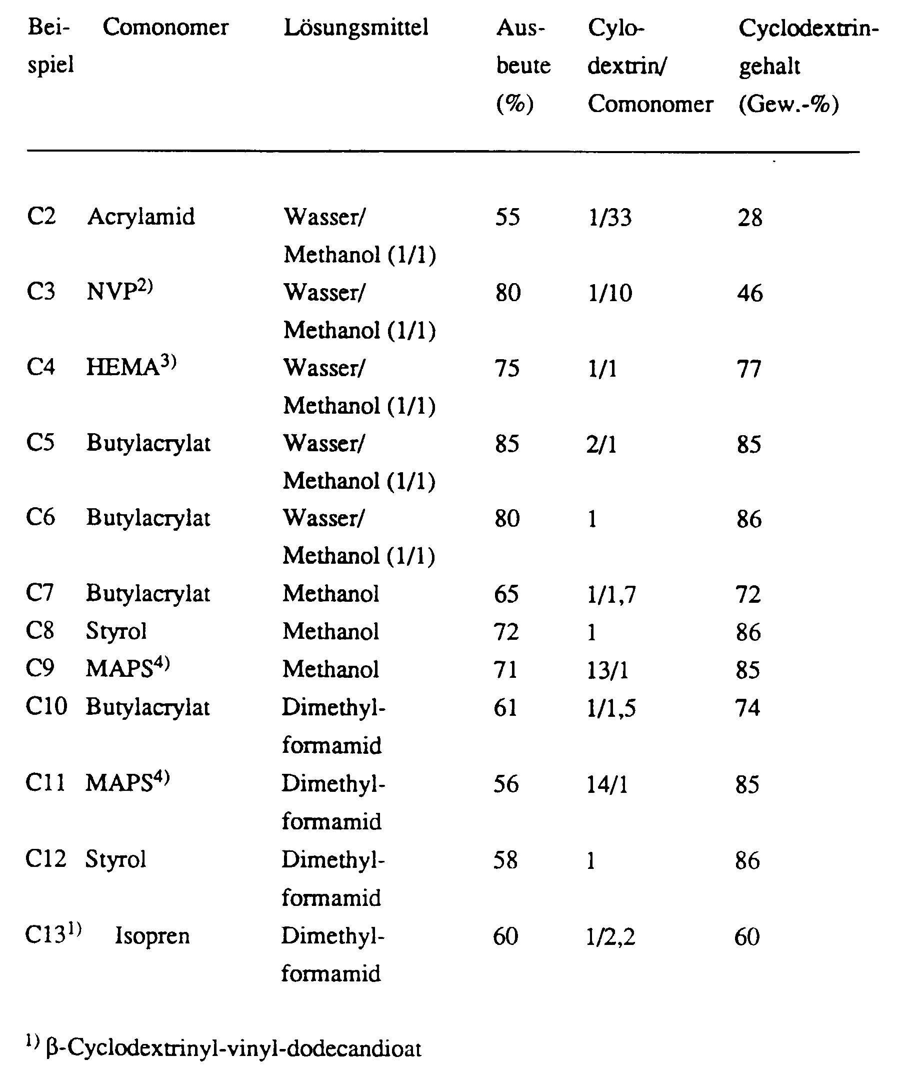

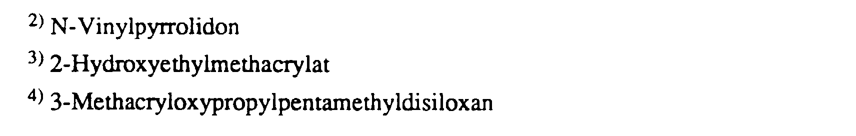

### Beispiel C14:

12 mg Azo-bis-isobutyronitril und 200 mg (0,18 mmol) α-Cyclodextrinyl-vinyladipat werden unter Argon in einen 5 ml-Kolben eingewogen und in 1 ml argonisiertem Methanol/-Wasser (1/1) gelöst. Hydroxyethylmethacrylat (0,36 mmol) und 10 µl Ethylenglykolbismethacrylat werden zugegeben und 18 h bei 60°C gerührt. In einer Ausbeute von 90 % wird ein unlösliches, transparentes Hydrogel erhalten.

## Patentansprüche

1. Verbindungen der Formeln I und Ia,
R-Y-CO-R₃-CO-O-A (I),
R-Y-CO-R₃-CO-O-CH₂-A₁ (Ia),
worin R eine radikalisch polymerisierbare Kohlenwasserstoffgruppe bedeutet, R₃ für eine direkte Bindung, lineares oder verzweigtes C₁-C₂₂-Alkylen C₃-C₈-Cycloalkylen oder C₆-C₁₄-Arylen steht, A für den in einer 2- oder 3-Stellung um eine Hydroxylgruppe verminderten Rest eines cyclisch-oligomeren Kohlenhydrats oder eines solchen Kohlenhydratderivats steht, A₁ den um eine Hydroxymethylgruppe verminderten Rest eines monomeren, oder linearen oder verzweigten oligomeren Kohlenhydrats oder eines solchen Kohlenhydratderivats bedeutet, und Y -O-, -NH- oder -N(C₁-C₆-Alkyl)- darstellt.

2. Verbindungen gemäss Anspruch 1, dadurch gekennzeichnet, dass R 2 bis 12 C-Atome enthält.

3. Verbindungen gemäss Anspruch 1, dadurch gekennzeichnet, dass der Rest R Ethen- oder Ethingruppen als radikalisch polymerisierbare Gruppen enthält.

4. Verbindungen gemäss Anspruch 1, dadurch gekennzeichnet, dass es sich bei R um Alkenyl, Alkinyl, Vinylphenyl oder Vinylbenzyl handelt.

5. Verbindungen gemäss Anspruch 4, dadurch gekennzeichnet, dass R als Alkenyl Vinyl, Allyl, 1-Propen-2-yl, 1-Buten-2- oder -3- oder -4-yl, 2-Buten-3-yl, die Isomeren von Pentenyl, Hexenyl, Octenyl, Decenyl oder Dodecenyl darstellt.

6. Verbindungen gemäss Anspruch 4, dadurch gekennzeichnet, dass R als Alkinyl HCC-CₘH₂ₘ- oder C₁-C₉-Alkyl-CC-CₘH₂ₘ- darstellt, worin m eine ganze Zahl von 1 bis 10 ist, und die Gesamtzahl der C-Atome 3 bis 12 beträgt.

7. Verbindungen gemäss Anspruch 6, dadurch gekennzeichnet, dass R Propargyl darstellt.

8. Verbindungen gemäss Anspruch 1, dadurch gekennzeichnet, dass Y -O-, -NH-, -NMethyl- oder -NEthyl- bedeutet.

9. Verbindungen gemäss Anspruch 8, dadurch gekennzeichnet, dass Y -O- oder -NH- darstellt.

10. Verbindungen gemäss Anspruch 1, dadurch gekennzeichnet, dass R₃ als Alkylen 2 bis 22 C-Atome enthält.

11. Verbindungen gemäss Anspruch 10, dadurch gekennzeichnet, dass R₃ als Alkylen 2 bis 20 C-Atome enthält.

12. Verbindungen gemäss Anspruch 11, dadurch gekennzeichnet, dass R₃ als Alkylen 4 bis 18 C-Atome enthält.

13. Verbindungen gemäss Anspruch 1, dadurch gekennzeichnet, dass R₃ als Cycloalkylen 4 bis 6 C-Atome enthält.

14. Verbindungen gemäss Anspruch 1, dadurch gekennzeichnet, dass R₃ als Arylen C₆-C₁₄-Arylen darstellt.

15. Verbindungen gemäss Anspruch 14, dadurch gekennzeichnet, dass R₃ 1,2-, 1,3- und 1,4-Phenylen, 2,3-, 2,7 oder 2,8-Naphthylen, und Biphenylene der Formel 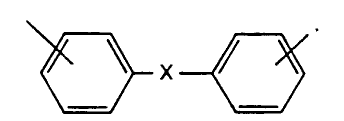 bedeutet, worin X eine direkte Bindung, -CH₂-, CH₃CH=, (CH₃)₂C=, Cyclohexyliden, -O-, -S-, -CO-, -CO₂-, -SO-, -SO₂-, -NH-, -CO-NH-, -N(C₁-C₄-Alkyl)- oder -CO-N(C₁-C₄-Alkyl)- darstellt.

16. Verbindungen gemäss Anspruch 1, dadurch gekennzeichnet, dass A als Rest eines cyclisch-oligomeren Kohlenhydrats ein Cyclodextrin mit 6 bis 8 gleichen oder verschiedenen Einheiten eines Monosaccharids bedeutet.

17. Verbindungen gemäss Anspruch 16, dadurch gekennzeichnet, dass sich A als Rest eines Cyclodextrins oder Cyclodextrinderivats von α-, β- oder γ-Cyclodextrin, oder 6-Methyl-, 6-Hydroxyethyl-, 6-Hydroxypropyl-, 6-Acetyl- oder 6-Maltosylcyclodextrin als Cyclodextrinderivat ableitet.

18. Verbindungen gemäss Anspruch 1, dadurch gekennzeichnet, dass sich A₁ als Rest eines oligomeren Kohlenhydrats von einem Oligosaccharid mit 2 bis 10 gleichen oder verschiedenen Saccharideinheiten ableitet.

19. Verbindungen gemäss Anspruch 1, dadurch gekennzeichnet, dass sich A₁ als Rest eines Mono- und Oligosaccharids von Aldosen oder Ketosen ableitet.

20. Verbindungen gemäss Anspruch 1, dadurch gekennzeichnet, dass sich A₁ als Rest eines Monosaccharids von Aldopentosen, Aldohexosen, Ketopentosen oder Ketohexosen ableitet.

21. Verbindungen gemäss Anspruch 1, dadurch gekennzeichnet, dass sich A₁ als Rest eines Monosaccharids von D-Ribose, D-Arabinose, D-Xylose oder D-Lyxose; D-Allose, D-Altrose, D-Glucose, D-Mannose, D-Gulose, D-Idose, D-Galaktose, D-Talose; D-Ribulose, D-Xylulose; -Psicose, D-Fructose, D-Sorbose oder D-Tagatose; als Rest eines Disaccharids von Trehalose, Maltose, Isomaltose, Cellobiose, Gentiobiose, Saccharose oder Lactose; und als Rest eines Trisaccharids von Raffinose oder Panose ableitet.

22. Verbindungen gemäss Anspruch 1, dadurch gekennzeichnet, dass es sich bei A₁ als Rest eines Oligosaccharids um ein oligomeres Abbauprodukt von Polysacchariden handelt.

23. Verbindungen gemäss Anspruch 1, dadurch gekennzeichnet, dass es sich bei A₁ als Rest von Derivaten monomerer und linearer oder verzweigter oligomerer Kohlenhydrate um solche handelt, die in 1- und/oder 2- und/oder 3-Stellung mit C₁-C₁₂-Alkyl, C₂-C₄-Hydroxyalkyl und C₁-C₁₂-Acyl substituiert sind, oder dass es sich um natürliche und synthetische Nukleoside sowie Oligonukleotide aus 2 bis 20 gleicher oder verschiedener solcher Nukleoside handelt.

24. Verbindungen gemäss Anspruch 1, dadurch gekennzeichnet, dass die Gruppe R Allyl, Propargyl, p-Vinylphenyl, p-Vinylbenzyl oder einen Rest der Formel R₁CH=CR₂- darstellt, worin R₁ H oder C₁-C₆-Alkyl bedeutet und R₂ H, C₁-C₆-Alkyl oder Phenyl darstellt.

25. Verbindungen gemäss Anspruch 24, dadurch gekennzeichnet, dass R₁ als Alkyl 1 bis 4 C-Atome enthält.

26. Verbindungen gemäss Anspruch 25, dadurch gekennzeichnet, dass R₁ als Alkyl Methyl oder Ethyl darstellt.

27. Verbindungen gemäss Anspruch 24, dadurch gekennzeichnet, dass R₁ H, Methyl oder Ethyl darstellt.

28. Verbindungen gemäss Anspruch 27, dadurch gekennzeichnet, dass R₁ H ist.

29. Verbindungen gemäss Anspruch 24, dadurch gekennzeichnet, dass R₂ als Alkyl 1 bis 4 C-Atome enthält.

30. Verbindungen gemäss Anspruch 24, dadurch gekennzeichnet, dass R₂ als Alkyl Methyl, Ethyl, n-Propyl n-Butyl, n- Pentyl und n-Hexyl bedeutet.

31. Verbindungen gemäss Anspruch 24, dadurch gekennzeichnet, dass R₂ H, Methyl, Ethyl, Propyl oder Butyl ist.

32. Verbindungen gemäss Anspruch 24, dadurch gekennzeichnet, dass R₁ H und R₂ H, Methyl, Ethyl, n-Propyl oder n-Butyl bedeutet.

33. Verbindungen gemäss Anspruch 24, dadurch gekennzeichnet, dass R₁ und R₂ je H darstellen.

34. Verfahren zur Herstellung der Verbindungen der Formeln I und Ia gemäss Anspruch 1, dadurch gekennzeichnet, dass man einen Divinyldicarbonsäureester der Formel II
R₁CH=CR₂-O-CO-R₃-CO-O-R₂C=CHR₁ (II),
oder einen Monovinylester der Formel IIa
R"-Y-CO-R₃-CO-O-R₂C=CHR₁ (IIa),
worin R₁ und R₂ die nachfolgenden und R₃ und Y die zuvor angegebenen Bedeutungen haben, (a) mit einem cyclisch-oligomeren Kohlehydrat der Formel A-OH oder dessen Derivaten umestert, worin A die zuvor angegebene Bedeutung hat, oder (b) in Gegenwart von lipolytischen Enzymen mit einem monomeren oder linearen oligomeren Kohlenhydrat der Formel A₁-CH₂OH oder deren Derivaten umestert, worin A₁ die zuvor angegebene Bedeutung hat, und (c) gegebenenfalls die erhaltenen Verbindungen der Formeln I und Ia, worin R eine radikalisch polymerisierbare Vinylestergruppe bedeutet, mit einem Alkohol der Formel R'-OH , einem Amin R'NH₂ oder einem Amin R'NHC₁-C₄-Alkyl umestert oder amidiert, wobei R' und R" unabhängig voneinander für eine nicht-vinylische radikalisch polymerisierbare Kohlenwasserstoffgruppe stehen.

35. Polymere, die bezogen auf das Polymer
i) 0,1 bis 100 Mol-% mindestens eines Strukturelements der Formeln III und/oder IIIa enthalten, 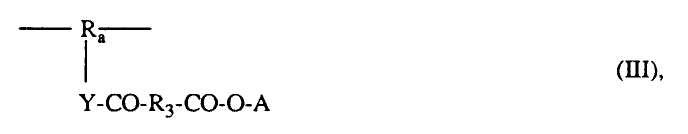 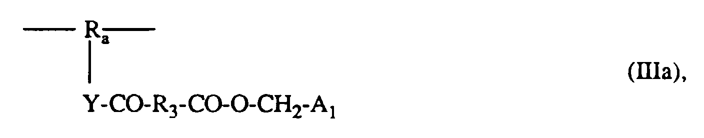 worin Rₐ den Rest einer radikalisch polymerisierten Gruppe darstellt und R₃, A, A₁ und Y die zuvor angegebenen Bedeutungen haben,
ii) 99,9 bis 0 Mol-% mindestens eines von den Formeln III und IIIa verschiedenen Strukturelements eines radikalisch polymerisierten Olefins, und
iii) 80 bis 0 Mol-% mindestens eines Strukturelements eines radikalisch polymerisierten Diolefins, wobei sich die Molprozente zu 100 % addieren.

36. Polymere gemäss Anspruch 35, dadurch gekennzeichnet, dass Rₐ 2 bis 12 C-Atome enthält.

37. Polymere gemäss Anspruch 35, dadurch gekennzeichnet, dass es sich bei dem Rest Rₐ um dreiwertige Ethantriyl-, Phenylen-ethylen-, Phenylenmethyl-ethylen- oder Ethentriyl-gruppen handelt.

38. Polymere gemäss Anspruch 37, dadurch gekennzeichnet, dass die Ethantriylgruppen unsubstituiert oder mit Alkyl substituiert sind, wobei die so substituierten Ethantriylgruppen bis zu 12 C-Atome enthalten.

39. Polymere gemäss Anspruch 37, dadurch gekennzeichnet, dass Rₐ Ethantriyl, Propan-1,2,3-triyl, Butan-1,2,4-triyl, Pentan-1,2,5-triyl, Hexan-1,2,6-triyl, Heptan-1,2,7-triyl, Octan-1,2,8-triyl, -CH₂-CH(C₆H₄-)- und -CH₂-CH(C₆H₄CH₂-)- bedeutet.

40. Polymere gemäss Anspruch 37, dadurch gekennzeichnet, dass es sich bei der Ethentriylgruppe um solche der Formeln -HC=C(CₘH₂ₘ-)- oder -(C₁-C₉-Alkyl)C=C(CₘH₂ₘ-)- handelt, worin m eine ganze Zahl von 1 bis 10 ist, und die Gesamtzahl der C-Atome 3 bis 12 beträgt.

41. Polymere gemäss Anspruch 40, dadurch gekennzeichnet, dass Rₐ -HC=C(CH₂-)- ist.

42. Polymere gemäss Anspruch 35, dadurch gekennzeichnet, dass Rₐ Reste der Formeln -CH₂-CH(CH₂-)-, -CH₂-CH(C₆H₄-)-, -CH₂-CH(C₆H₄CH₂-)-, -HC=C(CH₂-)- oder -(R₁)CH-C(R₂)= darstellt, worin R₁ H oder C₁-C₆-Alkyl bedeutet und R₂ H, C₁-C₆-Alkyl oder Phenyl darstellt.

43. Polymere gemäss Anspruch 35, dadurch gekennzeichnet, dass die Komponente i) in einer Menge von 0,5 bis 100 Mol-% enthalten ist.

44. Polymere gemäss Anspruch 35, dadurch gekennzeichnet, dass die Komponente ii) in einer Menge von 99,5 bis 0,5 Mol-% enthalten ist.

45. Polymere gemäss Anspruch 35, dadurch gekennzeichnet, dass die Komponente iii) in einer Menge von 70 bis 0,01 Mol-% enthalten ist.

46. Polymere gemäss Anspruch 35, dadurch gekennzeichnet, dass die Strukturelemente der Komponente i) den Formeln IV und/oder IVa entsprechen, 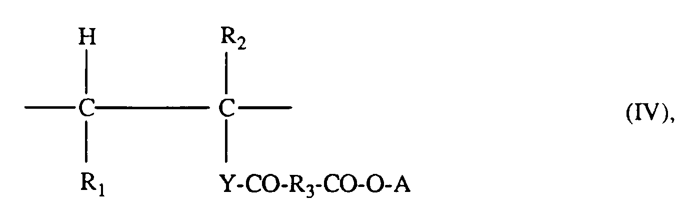 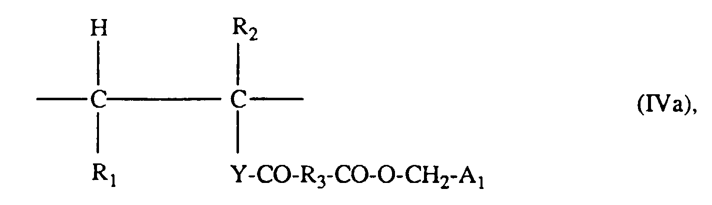 worin R₁ H oder C₁-C₆-Alkyl bedeutet und R₂ H, C₁-C₆-Alkyl oder Phenyl darstellt, und A, A₁, R₃ und Y die in Anspruch 35 angegebenen Bedeutungen haben.

47. Polymere gemäss Anspruch 46, dadurch gekennzeichnet, dass R₁ H und R₂ H, Methyl, Ethyl, n-Propyl oder n-Butyl sind.

48. Polymere gemäss Anspruch 46, dadurch gekennzeichnet, dass R₁ und R₂ je für H stehen.

49. Polymere gemäss Anspruch 35, dadurch gekennzeichnet, dass es sich bei den Olefinmonomeren, von denen sich die Strukturelemente der Komponente ii) ableiten, um Ethylen handelt, das unsubstituiert oder mit Halogen, -OH, -CN, Pyrrolidonyl, C₁-C₁₂-Alkyl, Phenyl, C₁-C₄-Alkylphenyl, C₁-C₄-Alkoxyphenyl, Halogenphenyl, Hydroxyphenyl, C₁-C₄-Alkyl-hydroxy-phenyl, C₁-C₄-Alkoxy-hydroxy-phenyl, Chlor- oder Brom-hydroxyphenyl, C₁-C₄-Alkoxy, Phenoxy, C₁-C₄-Alkylphenoxy, Benzyl, Benzyloxy, -COO^{⊖}M^{⊕} , -COOR₄, -COOCH₂CH(OH)CH₂OH, -COBR₅-OH, -COO-(R₆R₇SiO)ₙ-SiR₆R₇R₈, -COBR₅-O-(R₆R₇SiO)ₙ-SiR₆R₇R₈, -CONH₂, -CONH(C₁-C₆-Alkyl), -CON(C₁-C₆-Alkyl)₂ oder -OCO-R₄ substituiert ist, worin M^{⊕} für H^{⊕}, ein Alkalimetallkation oder ein Ammoniumkation steht, R₄ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₅ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, B für -O-, -N(C₁-C₆-Alkyl)- oder -NH- steht, R₆, R₇ und R₈ unabhängig voneinander C₁-C₆-Alkyl oder C₁-C₆-Alkoxy bedeuten und n für eine Zahl von 1 bis 30 steht.

50. Polymere gemäss Anspruch 49, dadurch gekennzeichnet, dass R₄ als Alkyl C₁-C₆-Alkyl ist.

51. Polymere gemäss Anspruch 49, dadurch gekennzeichnet, dass R₅ als Alkylen 2 bis 6 C-Atome enthält.

52. Polymere gemäss Anspruch 49, dadurch gekennzeichnet, dass R₆, R₇ und R₈ Methyl, Ethyl, Methoxy oder Ethoxy darstellen.

53. Polymere gemäss Anspruch 49, dadurch gekennzeichnet, dass es sich bei M^{⊕} in der Bedeutung eines Ammoniumkations um NH₄^{⊕} oder die Kationen eines primären Amins mit 1 bis 12 C-Atomen, eines sekundären Amins mit 2 bis 18 C-Atomen, eines tertiären Amins mit 3 bis 24 C-Atomen, oder um quaternäres Ammonium mit 4 bis 30, bevorzugt 4 bis 20 C-Atomen handelt.

54. Polymere gemäss Anspruch 49, dadurch gekennzeichnet, dass es sich bei Komponente ii) um Struktureinheiten der Formel V handelt, 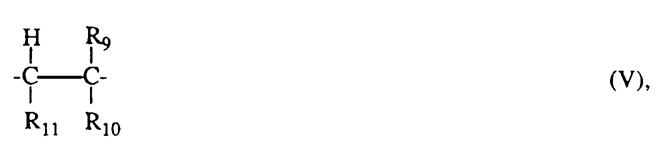 worin R₁₁ für H, C₁-C₆-Alkyl, -COOR₂₀ oder -COO^{⊖}M^{⊕} steht, R₉ H, F, Cl, CN oder C₁-C₆-Alkyl bedeutet, R₁₀ H, F, Cl, CN, Pyrrolidonyl, R₁₂O-, C₁-C₁₂-Alkyl, -OH, -COO^{⊖}M^{⊕}, -COOR₄, -COOCH₂CH(OH)CH₂OH, -CONH₂, -CONH(C₁-C₄-Alkyl), -CON(C₁-C₄-Alkyl)₂, -COBR₅-OH, -OCO-R₄, -COO-(R₆R₇SiO)ₙ-SiR₆R₇R₈, -COBR₅-O-(R₆R₇SiO)ₙ-SiR₆R₇R₈, Phenyl oder mit -OH und/oder ein oder zwei Methyl, Methoxy, Cl oder Br substituiertes Phenyl darstellt, M^{⊕} für H^{⊕} , ein Alkalimetallkation oder ein Ammoniumkation steht, R₄ C₁-C₁₈-Alkyl, C₅-C₇-Cycloalkyl, (C₁-C₁₂-Alkyl)-C₅-C₇-cycloalkyl, Phenyl, (C₁-C₁₂-Alkyl)phenyl, Benzyl oder (C₁-C₁₂-Alkyl)benzyl darstellt, R₁₂ für lineares oder verzweigtes C₂-C₁₈-Alkylen, Poly(C₂-C₆-oxaalkylen) mit 2 bis 6 Oxaalkyleneinheiten, C₅-C₈-Cycloalkylen, Phenylen, Benzylen oder Xylylen steht, B für -O-, -N(C₁-C₄-Alkyl)- oder -NH- steht, und R₆, R₇ und R₈ Methyl, Ethyl, Methoxy oder Ethoxy bedeuten, und n für 0 oder eine Zahl von 1 bis 20, bevorzugt 1 bis 12 bedeutet.

55. Polymere gemäss Anspruch 54, dadurch gekennzeichnet, dass R₁₁ für H steht.

56. Polymere gemäss Anspruch 54, dadurch gekennzeichnet, dass R₉ für H, Cl oder C₁-C₄-Alkyl steht.

57. Polymere gemäss Anspruch 54, dadurch gekennzeichnet, dass R₁₁ für H steht, R₉ für H, F, Cl, Methyl oder Ethyl steht, und R₁₀ für Pyrrolidonyl, -F, -Cl, -CN, -OH, C₁-C₄-Alkyl, C₁-C₆-Alkoxy, -COO-C₁-C₆-Alkyl, -COO-R₅-OH, -COOM^{⊕}, -OOC-C₁-C₆-Alkyl, -COOCH₂CH(OH)CH₂OH, -CONH₂, -CONH(C₁-C₄-Alkyl), -CON(C₁-C₄-Alkyl)₂, -COO-(R₆R₇SiO)ₙ-SiR₆R₇R₈, -COBR₅-O-(R₆R₇SiO)ₙ-SiR₆R₇R₈, Phenyl, Methylphenyl, Dimethylphenyl, Chlorphenyl, Dichlorphenyl, Dibromphenyl, Methoxyphenyl, Dimethoxyphenyl, Hydroxyphenyl, worin M^{⊕} Trialkylammonium mit 1 bis 4 C-Atomen in den Alkylgruppen bedeutet, und R₅ C₂-C₆-Alkylen darstellt, und R₆, R₇ und R₈ Methyl, Ethyl, Methoxy oder Ethoxy bedeuten, und n für 0 oder eine Zahl von 1 bis 12 bedeutet.

58. Polymere gemäss Anspruch 54, dadurch gekennzeichnet, dass in Formel V R₁₁ für H, R₉ für H oder Methyl und R₁₀ für Pyrridonyl, -CN, -COOH, -COO-C_{y}H_{2y}-OH mit y gleich 2 bis 6, -CON(CH₃)₂, -COO-CH₂CH(OH)CH₂OH und -COO-CH₂CH₂-O-[Si(OCH₃)₂-O]ₙ-Si(OCH₃)₃ oder -COO-CH₂CH₂-O-[Si(OC₂H₅)₂-O]ₙ-Si(OC₂H₅)₃ mit n gleich 1 bis 8 und bevorzugt 2 bis 6 stehen.

59. Polymere gemäss Anspruch 35, dadurch gekennzeichnet, dass es sich bei der Komponente iii) um Struktureinheiten von Butadien, Isopren und Chloropren handelt.

60. Polymere gemäss Anspruch 35, dadurch gekennzeichnet, dass es sich bei der Komponente iii) um Struktureinheiten von Diacrylaten oder Dimethacrylaten von Diolen oder von Diacryl- oder Dimethacrylamiden von Diaminen ableiten.

61. Polymere gemäss Anspruch 60, dadurch gekennzeichnet, dass es sich bei den Diolen und Diaminen um solche der Formel HY-CₓH₂ₓ-YH handelt, worin x für eine Zahl von 2 bis 12 steht, und Y -O-, -NH- oder -N(C₁-C₄-Alkyl)- bedeutet, oder um Polyoxaalkylendiole der Formel HO-(CₘH₂ₘO)_{y}-H handelt, worin m für eine Zahl von 2 bis 6 und y für eine Zahl von 2 bis 20 stehen.

62. Kontaktlinse enthaltend ein Polymer gemäss Anspruch 35.

63. Kontaktlinse bestehend aus einem Polymer gemäss Anspruch 35.

64. Kontaktlinse, die eine äussere Schicht aus einem Polymer gemäss Anspruch 35 aufweist.

65. Polymere gemäss Anspruch 35, dadurch gekennzeichnet, dass sie als vierte Komponente iiii) bis zu 10 Mol-% Reste von trifunktionellen ethylenisch ungesättigten Verbindungen enthalten, wobei sich die Molprozente zu 100 % addieren.

66. Verwendung der wasserlöslichen Polymeren gemäss Anspruch 35 als Verdickungsmittel und Tenside.

67. Verwendung der Polymere gemäss Anspruch 35 als Trägermaterial für Wirkstoffe für eine kontrollierte Wirkstoffabgabe.

68. Verwendung der Polymere gemäss Anspruch 35 zur Herstellung, Modifizierung oder Reinigung von Kontaktlinsen.

## Claims

1. A compound of formula I or Ia
R-Y-CO-R₃-CO-O-A (I),
R-Y-CO-R₃-CO-O-CH₂-A₁ (Ia),
wherein
R is a radically polymerisable hydrocarbon group,
R₃ is a direct bond, linear or branched C₁-C₂₂alkylene, C₃-C₈cycloalkylene or C₆-C₁₄-arylene,
A is the radical, reduced by a hydroxy group in a 2- or 3-position, of a cyclic-oligomeric carbohydrate or of a derivative of such a carbohydrate,
A₁ is the radical, reduced by a hydroxymethyl group, of a monomeric or linear or branched oligomeric carbohydrate or of a derivative of such a carbohydrate, and
Y is -O-, -NH- or -N(C₁-C₆alkyl)-.

2. A compound according to claim 1 wherein R contains from 2 to 12 carbon atoms.

3. A compound according to claim 1 wherein the radical R contains ethene or ethyne groups as radically polymerisable groups.

4. A compound according to claim 1 wherein R is alkenyl, alkynyl, vinylphenyl or vinylbenzyl.

5. A compound according to claim 4 wherein R as alkenyl is vinyl, allyl, 1-propen-2-yl, 1-buten-2- or -3- or -4-yl, 2-buten-3-yl, or an isomer of pentenyl, hexenyl, octenyl, decenyl or dodecenyl.

6. A compound according to claim 4 wherein R as alkynyl is HCC-CₘH₂ₘ- or C₁-C₉alkyl-CC-CₘH₂ₘ- wherein m is an integer from 1 to 10 and the total number of carbon atoms is from 3 to 12.

7. A compound according to claim 6 wherein R is propargyl.

8. A compound according to claim 1 wherein Y is -O-, -NH-, -Nmethyl- or -Nethyl-.

9. A compound according to claim 8 wherein Y is -O- or -NH-.

10. A compound according to claim 1 wherein R₃ as alkylene contains from 2 to 22 carbon atoms.

11. A compound according to claim 10 wherein R₃ as alkylene contains from 2 to 20 carbon atoms.

12. A compound according to claim 11 wherein R₃ as alkylene contains from 4 to 18 carbon atoms.

13. A compound according to claim 1 wherein R₃ as cycloalkylene contains from 4 to 6 carbon atoms.

14. A compound according to claim 1 wherein R₃ as arylene is C₆-C₁₄arylene.

15. A compound according to claim 14 wherein R₃ is 1,2-, 1,3- or 1,4-phenylene, 2,3-, 2,7-or 2,8-naphthylene, or a biphenylene of the formula 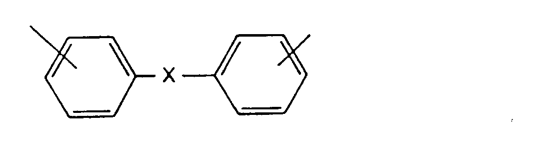 wherein X is a direct bond, -CH₂-, CH₃CH=, (CH₃)₂C=, cyclohexylidene, -O-, -S-, -CO-, -CO₂-, -SO-, -SO₂-, -NH-, -CO-NH-, -N(C₁-C₄alkyl)- or -CO-N(C₁-C₄alkyl)-.

16. A compound according to claim 1 wherein A as the radical of a cyclic-oligomeric carbohydrate is a cyclodextrin having from 6 to 8 identical or different monosaccharide units.

17. A compound according to claim 16 wherein A as the radical of a cyclodextrin or cyclodextrin derivative is derived from α-, β- or γ-cyclodextrin or from 6-methyl-, 6-hydroxyethyl-, 6-hydroxypropyl-, 6-acetyl- or 6-maltosyl-cyclodextrin as cyclodextrin derivative.

18. A compound according to claim 1 wherein A₁ as the radical of an oligomeric carbohydrate is derived from an oligosaccharide having from 2 to 10 identical or different saccharide units.

19. A compound according to claim 1 wherein A₁ as the radical of a mono- or oligosaccharide is derived from an aldose or ketose.

20. A compound according to claim 1 wherein A₁ as the radical of a monosaccharide is derived from an aldopentose, aldohexose, ketopentose or ketohexose.

21. A compound according to claim 1 wherein A₁ as the radical of a monosaccharide is derived from D-ribose, D-arabinose, D-xylose or D-lyxose; D-allose, D-altrose, D-glucose, D-mannose, D-gulose, D-idose, D-galactose or D-talose; D-ribulose or D-xylulose; D-psicose, D-fructose, D-sorbose or D-tagatose; as the radical of a disaccharide is derived from trehalose, maltose, isomaltose, cellobiose, gentiobiose, saccharose or lactose; and as the radical of a trisaccharide is derived from raffinose or panose.

22. A compound according to claim 1 wherein A₁ as the radical of an oligosaccharide is an oligomeric decomposition product of a polysaccharide.

23. A compound according to claim 1 wherein when A₁ is the radical of derivatives of monomeric and linear or branched oligomeric carbohydrates, those derivatives are substituted in the 1- and/or 2- and/or 3-position(s) by C₁-C₁₂alkyl, C₂-C₄hydroxyalkyl and C₁-C₁₂acyl, or are natural and synthetic nucleosides and also oligonucleotides comprising from 2 to 20 such nucleosides which may be identical or different.

24. A compound according to claim 1 wherein the group R is allyl, propargyl, p-vinylphenyl, p-vinylbenzyl or a radical of the formula R₁CH=CR₂- wherein R₁ is H or C₁-C₆-alkyl and R₂ is H, C₁-C₆alkyl or phenyl.

25. A compound according to claim 24 wherein R₁ as alkyl contains from I to 4 carbon atoms.

26. A compound according to claim 25 wherein R₁ as alkyl is methyl or ethyl.

27. A compound according to claim 24 wherein R₁ is H, methyl or ethyl.

28. A compound according to claim 27 wherein R₁ is H.

29. A compound according to claim 24 wherein R₂ as alkyl contains from 1 to 4 carbon atoms.

30. A compound according to claim 24 wherein R₂ as alkyl is methyl, ethyl, n-propyl, n-butyl, n-pentyl or n-hexyl.

31. A compound according to claim 24 wherein R₂ is H, methyl, ethyl, propyl or butyl.

32. A compound according to claim 24 wherein R₁ is H and R₂ is H, methyl, ethyl, n-propyl or n-butyl.

33. A compound according to claim 24 wherein R₁ and R₂ are each H.

34. A process for the preparation of a compound of formula I or Ia according to claim 1, wherein a divinyldicarboxylic acid ester of formula II
R₁CH=CR₂-O-CO-R₃-CO-O-R₂C=CHR₁ (II),
or a monovinyl ester of formula IIa
R"-Y-CO-R₃-CO-O-R₂C=CHR₁ (IIa),
wherein R₁ and R₂ are as defined below and R₃ and Y are as defined above, is
(a) transesterified with a cyclic-oligomeric carbohydrate of the formula A-OH, or with a derivative thereof, wherein A is as defined above, or
(b) transesterified in the presence of lipolytic enzymes with a monomeric or linear oligomeric carbohydrate of the formula A₁-CH₂OH, or with a derivative thereof, wherein A₁ is as defined above, and
(c) if desired the resulting compound of formula I or Ia wherein R is a radically polymerisable vinyl ester group is transesterified or amidated with an alcohol of the formula R'-OH, with an amine R'NH₂ or with an amine R'NHC₁-C₄alkyl, with R' and R" each independently of the other being a non-vinylic radically polymerisable hydrocarbon group.

35. A polymer that, based on the polymer, comprises
i) 0.1 to 100 mol % of at least one structural element of formula III and/or IIIa 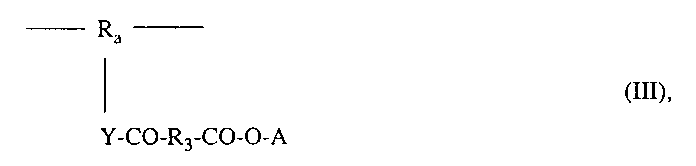 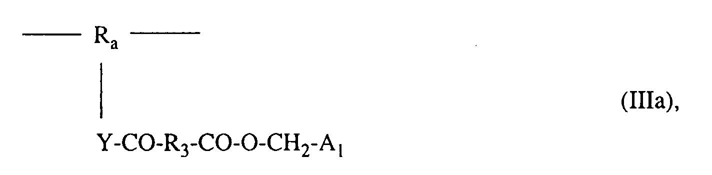 wherein Rₐ is the radical of a radically polymerised group and R₃, A, A₁ and Y are as defined above,
ii) 99.9 to 0 mol % of at least one structural element, different from formulae III and IIIa, of a radically polymerised olefin, and
iii) 80 to 0 mol % of at least one structural element of a radically polymerised diolefin, the molar percentages totalling 100 %.

36. A polymer according to claim 35 wherein Rₐ contains from 2 to 12 carbon atoms.

37. A polymer according to claim 35 wherein the radical Rₐ is a trivalent ethanetriyl, phenylene-ethylene, phenylenemethyl-ethylene or ethenetriyl group.

38. A polymer according to claim 37 wherein the ethanetriyl group is unsubstituted or substituted by alkyl, the ethanetriyl group so substituted containing up to 12 carbon atoms.

39. A polymer according to claim 37 wherein Rₐ is ethanetriyl, propane-1,2,3-triyl, butane-1,2,4-triyl, pentane-1,2,5-triyl, hexane-1,2,6-triyl, heptane-1,2,7-triyl, octane-1,2,8-triyl, -CH₂-CH(C₆H₄-)- or -CH₂-CH(C₆H₄CH₂-)-.

40. A polymer according to claim 37 wherein the ethenetriyl group is a group of the formula -HC=C(CₘH₂ₘ-)- or -(C₁-C₉alkyl)C=C(CₘH₂ₘ-)- wherein m is an integer from 1 to 10 and the total number of carbon atoms is from 3 to 12.

41. A polymer according to claim 40 wherein Rₐ is -HC=C(CH₂-)-.

42. A polymer according to claim 35 wherein Rₐ is a radical of the formula -CH₂-CH(CH₂-)-, -CH₂-CH(C₆H₄-)-, -CH₂-CH(C₆H₄CH₂-)-, -HC=C(CH₂-)- or -(R₁)CH-C(R₂)= wherein R₁ is H or C₁-C₆alkyl and R₂ is H, C₁-C₆alkyl or phenyl.

43. A polymer according to claim 35 wherein component i) is present in an amount of from 0.5 to 100 mol %.

44. A polymer according to claim 35 wherein component ii) is present in an amount of from 99.5 to 0.5 mol %.

45. A polymer according to claim 35 wherein component iii) is present in an amount of from 70 to 0.01 mol %.

46. A polymer according to claim 35 wherein the structural elements of component i) correspond to formula IV and/or IVa 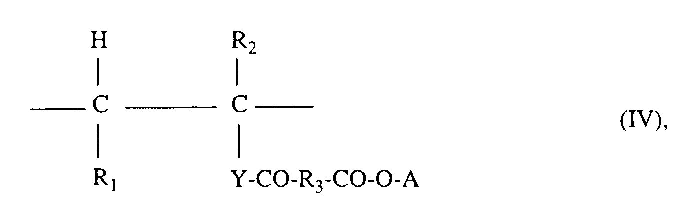 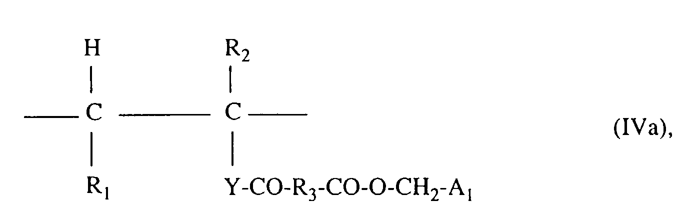 wherein R₁ is H or C₁-C₆alkyl and R₂ is H, C₁-C₆alkyl or phenyl, and A, A₁, R₃ and Y are as defined in claim 35.

47. A polymer according to claim 46 wherein R₁ is H and R₂ is H, methyl, ethyl, n-propyl or n-butyl.

48. A polymer according to claim 46 wherein R₁ and R₂ are each H.

49. A polymer according to claim 35 wherein the olefin monomers from which the structural elements of component ii) are derived are ethylene which is unsubstituted or substituted by halogen, -OH, -CN, pyrrolidonyl, C₁-C₁₂alkyl, phenyl, C₁-C₄alkylphenyl, C₁-C₄alkoxyphenyl, halophenyl, hydroxyphenyl, C₁-C₄alkylhydroxyphenyl, C₁-C₄alkoxyhydroxyphenyl, chloro- or bromo-hydroxyphenyl, C₁-C₄alkoxy, phenoxy, C₁-C₄alkylphenoxy, benzyl, benzyloxy, -COO^{⊖}M^{⊕}, -COOR₄, -COOCH₂CH(OH)CH₂OH, -COBR₅-OH, -COO-(R₆R₇SiO)ₙ-SiR₆R₇R₈, -COBR₅-O-(R₆R₇SiO)ₙ-SiR₆R₇R₈, -CONH₂, -CONH(C₁-C₆alkyl), -CON(C₁-C₆alkyl)₂ or by -OCO-R₄ wherein M^{⊕} is H^{⊕}, an alkali metal cation or an ammonium cation, R₄ is C₁-C₁₈alkyl, C₅-C₇cycloalkyl, (C₁-C₁₂alkyl)-C₅-C₇cycloalkyl, phenyl, (C₁-C₁₂alkyl)phenyl, benzyl or (C₁-C₁₂alkyl)benzyl, R₅ is linear or branched C₂-C₁₈alkylene, poly(C₂-C₆oxaalkylene) having from 2 to 6 oxaalkylene units, C₅-C₈cycloalkylene, phenylene, benzylene or xylylene, B is -O-, -N(C₁-C₆alkyl)- or -NH-, R₆, R₇ and R₈ are each independently of the other C₁-C₆alkyl or C₁-C₆alkoxy and n is a number from 1 to 30.

50. A polymer according to claim 49 wherein R₄ as alkyl is C₁-C₆alkyl.

51. A polymer according to claim 49 wherein R₅ as alkylene contains from 2 to 6 carbon atoms.

52. A polymer according to claim 49 wherein R₆, R₇ and R₈ are methyl, ethyl, methoxy or ethoxy.

53. A polymer according to claim 49 wherein M^{⊕} as an ammonium cation is NH₄^{⊕} or the cation of a primary amine having from I to 12 carbon atoms, of a secondary amine having from 2 to 18 carbon atoms, or of a tertiary amine having from 3 to 24 carbon atoms, or is quaternary ammonium having from 4 to 30, preferably from 4 to 20, carbon atoms.

54. A polymer according to claim 49 wherein component ii) comprises structural units of formula V 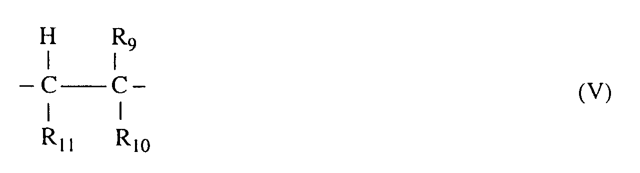 wherein
R₁₁ is H, C₁-C₆alkyl, -COOR₂₀ or -COO^{⊖}M^{⊕},
R₉ is H, F, Cl, CN or C₁-C₆alkyl,
R₁₀ is H, F, Cl, CN, pyrrolidonyl, R₁₂O-, C₁-C₁₂alkyl, -OH, -COO^{⊖}M^{⊕}, -COOR₄, -COOCH₂CH(OH)CH₂OH, -CONH₂, -CONH(C₁-C₄alkyl), -CON(C₁-C₄alkyl)₂, -COBR₅-OH, -OCO-R₄, -COO-(R₆R₇SiO)ₙ-SiR₆R₇R₈, -COBR₅-O-(R₆R₇SiO)ₙ-SiR₆R₇R₈, phenyl, or phenyl substituted by -OH and/or by one or two methyl, methoxy, Cl or Br,
M^{⊕} is H^{⊕}, an alkali metal cation or an ammonium cation,
R₄ is C₁-C₁₈alkyl, C₅-C₇cycloalkyl, (C₁-C₁₂alkyl)-C₅-C₇cycloalkyl, phenyl, (C₁-C₁₂-alkyl)phenyl, benzyl or (C₁-C₁₂alkyl)benzyl,
R₁₂ is linear or branched C₂-C₁₈alkylene, poly(C₂-C₆oxaalkylene) having from 2 to 6 oxaalkylene units, C₅-C₈cycloalkylene, phenylene, benzylene or xylylene,
B is -O-, -N(C₁-C₄alkyl)- or -NH-,
R₆, R₇ and R₈ are methyl, ethyl, methoxy or ethoxy, and
n is 0 or a number from 1 to 20, preferably from 1 to 12.

55. A polymer according to claim 54 wherein R₁₁ is H.

56. A polymer according to claim 54 wherein R₉ is H, Cl or C₁-C₄alkyl.

57. A polymer according to claim 54 wherein R₁₁ is H, R₉ is H, F, Cl, methyl or ethyl, and R₁₀ is pyrrolidonyl, -F, -Cl, -CN, -OH, C₁-C₄alkyl, C₁-C₆alkoxy, -COO-C₁-C₆alkyl, -COO-R₅-OH, -COOM^{⊕}, -OOC-C₁-C₆alkyl, -COOCH₂CH(OH)CH₂OH, -CONH₂, -CONH(C₁-C₄alkyl), -CON(C₁-C₄alkyl)₂, -COO-(R₆R₇SiO)ₙ-SiR₆R₇R₈, -COBR₅-O-(R₆R₇SiO)ₙ-SiR₆R₇R₈, phenyl, methylphenyl, dimethylphenyl, chlorophenyl, dichlorophenyl, dibromophenyl, methoxyphenyl, dimethoxyphenyl or hydroxyphenyl wherein M^{⊕} is trialkylammonium having from 1 to 4 carbon atoms in the alkyl groups and R₅ is C₂-C₆-alkylene, and R₆, R₇ and R₈ are methyl, ethyl, methoxy or ethoxy, and n is 0 or a number from 1 to 12.

58. A polymer according to claim 54 wherein in formula V R₁₁ is H, R₉ is H or methyl, and R₁₀ is pyrrolidonyl, -CN, -COOH, -COO-C_{y}H_{2y}-OH wherein y is from 2 to 6, -CON(CH₃)₂, -COO-CH₂CH(OH)CH₂OH and -COO-CH₂CH₂-O-[Si(OCH₃)₂-O]ₙ-Si(OCH₃)₃ or -COO-CH₂CH₂-O-[Si(OC₂H₅)₂-O]ₙ-Si(OC₂H₅)₃ wherein n is from 1 to 8 and preferably from 2 to 6.

59. A polymer according to claim 35 wherein component iii) comprises structural units of butadiene, isoprene and chloroprene.

60. A polymer according to claim 35 wherein component iii) comprises structural units derived from diacrylates or dimethacrylates of diols or from diacrylamides or dimethacrylamides of diamines.

61. A polymer according to claim 60 wherein the diols and diamines are those of the formula HY-CₓH₂ₓ-YH wherein x is a number from 2 to 12, and Y is -O-, -NH- or -N(C₁-C₄alkyl)-, or polyoxaalkylenediols of the formula HO-(CₘH₂ₘO)_{y}-H wherein m is a number from 2 to 6 and y is a number from 2 to 20.

62. A contact lens comprising a polymer according to claim 35.

63. A contact lens consisting of a polymer according to claim 35.

64. A contact lens having an outer layer of a polymer according to claim 35.

65. A polymer according to claim 35 comprising as fourth component iiii) up to 10 mol % of radicals of trifunctional ethylenically unsaturated compounds, the molar percentages totalling 100 %.

66. The use of a water-soluble polymer according to claim 35 as a thickener or surfactant.

67. The use of a polymer according to claim 35 as a carrier for active ingredients to provide controlled release of the active ingredient.

68. The use of a polymer according to claim 35 in the manufacture, modification or cleaning of contact lenses.

## Revendications

1. Composés de formules I et Ia :
R-Y-CO-R₃-CO-O-A (I)
R-Y-CO-R₃-CO-O-CH₂A₁ (Ia)
dans lesquelles R représente un groupe hydrocarboné apte à une polymérisation radicalaire, R₃ représente une liaison directe, un groupe alkylène à chaîne droite ou ramifiée en C₁-C₂₂, un groupe cycloalkylène en C₃-C₈ ou arylène en C₆-C₁₄, A est le radical, diminué d'un groupe hydroxy en position 2 ou 3, d'un hydrate de carbone oligomère cyclique ou d'un dérivé d'un tel hydrate de carbone, A₁ représente le radical, diminué d'un groupe hydroxyméthyle, d'un hydrate de carbone monomère ou oligomère linéaire ou ramifié ou d'un dérivé d'un tel hydrate de carbone, et Y représente -O-, -NH- ou -N-(alkyle en C₁-C₆)-.

2. Composés selon la revendication 1, caractérisés en ce que R contient deux à douze atomes de carbone.

3. Composés selon la revendication 1, caractérisés en ce que R contient, en tant que groupes aptes à une polymérisation radicalaire, des groupes éthène ou éthyne.

4. Composés selon la revendication 1, caractérisés en ce que R est un groupe alcényle, alcynyle, vinylphényle ou vinylbenzyle.

5. Composés selon la revendication 4, caractérisés en ce que R est un groupe alcényle, à savoir un groupe vinyle, allyle, 1-propén-2-yle, 1-butén-2- ou -3- ou -4- yle, 2-butén-3-yle ou l'un des isomères des groupes pentényle, hexényle, octényle, décényle ou dodécényle.

6. Composés selon la revendication 4, caractérisés en ce que R est un groupe alcynyle, à savoir un groupe HCC-CₘH₂ₘ- ou (alkyle en C₁-C₉)-CC-CₘH₂ₘ- dans lequel m est un nombre entier allant de 1 à 10 et le nombre total des atomes de carbone est de trois à douze.

7. Composés selon la revendication 6, caractérisés en ce que R est un groupe propargyle.

8. Composés selon la revendication 1, caractérisés en ce que Y représente -O-, -NH-,-N-méthyle-ou -N-éthyle-.

9. Composés selon la revendication 8, caractérisés en ce que Y représente -O- ou -NH-.

10. Composés selon la revendication 1, caractérisés en ce que R₃ est un groupe alkylène en C₂-C₂₂.

11. Composés selon la revendication 10, caractérisés en ce que R₃ est un groupe alkylène en C₂-C₂₀.

12. Composés selon la revendication 11, caractérisés en ce que R₃ est un groupe alkylène en C₄-C₁₈.

13. Composés selon la revendication 1, caractérisés en ce que R₃ est un groupe cycloalkylène en C₄-C₆.

14. Composés selon la revendication 1, caractérisés en ce que R₃ est un groupe arylène en C₆-C₁₄.

15. Composés selon la revendication 14, caractérisés en ce que R₃ représente un groupe 1,2-, 1,3- ou 1,4-phénylène, 2,3-, 2,7- ou 2,8-naphtylène, ou biphénylène de formule 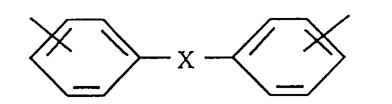 dans laquelle X représente une liaison directe, -CH₂-, CH₃CH=, (CH₃)₂C=, cyclohexylidène, -O-, -S-, -CO-, -CO₂-, -SO-, -SO₂-, -NH-, -CO-NH-, -N(alkyle en C₁-C₄)- ou -CO-N(alkyle en C₁-C₄)-.

16. Composés selon la revendication 1, caractérisés en ce que A, radical d'un hydrate de carbone oligomère cyclique, est un radical de cyclodextrine contenant six à huit motifs identiques ou différents de monosaccharides.

17. Composés selon la revendication 16, caractérisés en ce que A, radical d'une cyclodextrine ou d'un dérivé de cyclodextrine, dérive d'une α-, d'une β- ou d'une γ-cyclodextrine ou de la 6-méthyl-, de la 6-hydroxyéthyl-, de la 6-hydroxypropyl-, de la 6-acétyl- ou de la 6-maltosylcyclodextrine.

18. Composés selon la revendication 1, caractérisés en ce que A₁, radical d'un hydrate de carbone oligomère, dérive d'un oligosaccharide contenant deux à dix motifs identiques ou différents de saccharides.

19. Composés selon la revendication 1, caractérisés en ce que A₁, radical d'un mono ou d'un oligo-saccharide, dérive d'un aldose ou d'un cétose.

20. Composés selon la revendication 1, caractérisés en ce que A₁, radical d'un monosaccharide, dérive d'un aldopentose, d'un aldohexose, d'un cétopentose ou d'un cétohexose.

21. Composés selon la revendication 1, caractérisés en ce que A₁, radical d'un monosaccharide, dérive du D-ribose, du D-arabinose, du D-xylose ou du D-lyxose ; du D-allose, du D-altrose, du D-glucose, du D-mannose, du D-gulose, du D-idose, D-galactose, du D-talose ; du D-ribulose, du D-xylulose ; du D-psicose, du D-fructose, du D-sorbose ou du D-tagatose ; radical d'un disaccharide, il dérive du tréhalose, du maltose, de l'isomaltose, du cellobiose, du gentiobiose, du saccharose ou du lactose ; et radical d'un trisaccharide, il dérive du raffinose ou du panose.

22. Composés selon la revendication 1, caractérisés en ce que A₁, radical d'un oligosaccharide, consiste en un produit de dégradation oligomère d'un polysaccharide.

23. Composés selon la revendication 1, caractérisés en ce que A₁, radical d'un dérivé d'hydrate de carbone monomère ou oligomère linéaire ou ramifié, consiste en un radical substitué en positions 1 et/ou 2 et/ou 3 par des groupes alkyle en C₁-C₁₂, hydroxyalkyle en C₂-C₄ et acyle en C₁-C₁₂, ou en un radical de nucléoside naturel ou synthétique ou en un radical d'oligonucléotide contenant lui-même deux à vingt nucléosides identiques ou différents.

24. Composés selon la revendication 1, caractérisés en ce que R représente un groupe allyle, propargyle, p-vinylphényle, p-vinylbenzyle ou un groupe de formule R₁CH=CR₂- dans laquelle R₁ représente H ou un groupe alkyle en C₁-C₆ et R₂ représente H, un groupe alkyle en C₁-C₆ ou phényle.

25. Composés selon la revendication 24, caractérisés en ce que R₁ est un groupe alkyle en C₁-C₄.

26. Composés selon la revendication 25, caractérisés en ce que R₁, groupe alkyle, consiste en un groupe méthyle ou éthyle.

27. Composés selon la revendication 24, caractérisés en ce que R₁ représente H, un groupe méthyle ou éthyle.

28. Composés selon la revendication 27, caractérisés en ce que R₁ représente H.

29. Composés selon la revendication 24, caractérisés en ce que R₂ représente un groupe alkyle en C₁-C₄.

30. Composés selon la revendication 24, caractérisés en ce que R₂, groupe alkyle, consiste en un groupe méthyle, éthyle, n-propyle, n-butyle, n-pentyle ou n-hexyle.

31. Composés selon la revendication 24, caractérisés en ce que R₂ représente H, un groupe méthyle, éthyle, propyle ou butyle.

32. Composés selon la revendication 24, caractérisés en ce que R₁ représente H et R₂ représente H, un groupe méthyle, éthyle, n-propyle ou n-butyle.

33. Composés selon la revendication 24, caractérisés en que R₁ et R₂ représentent tous deux H.

34. Procédé de préparation des composés de formules I et Ia de la revendication 1, caractérisé en ce que l'on transestérifie un ester divinylique d'acide dicarboxylique de formule II
R₁CH=CR₂-O-CO-R₃-CO-O-R₂C=CHR₁ (II)
ou un ester monovinylique de formule IIa
R"-Y-CO-R₃-CO-O-R₂C=CHR₁ (IIa)
dans lesquelles R₁ et R₂ ont les significations indiquées ci-après et R₃ et Y les significations indiquées ci-dessus, (a) par un hydrate de carbone oligomère cyclique de formule A-OH ou par l'un de ses dérivés, A ayant les significations indiquées ci-dessus, ou bien (b) par un hydrate de carbone monomère ou oligomère linéaire de formule A₁-CH₂OH ou l'un de ses dérivés en présence d'enzymes lipolytiques, A₁ ayant les significations indiquées ci-dessus, et (c) le cas échéant, on transestérifie ou on amide les composés obtenus répondant aux formules I et la dans lesquelles R représente un groupe ester vinylique apte à une polymérisation radicalaire, par un alcool de formule R'-OH, une amine R'NH₂ ou une amine R'NH-alkyle en C₁-C₄, R' et R" représentant chacun, indépendamment l'un de l'autre, un groupe hydrocarboné non vinylique, apte ci une polymérisation radicalaire.

35. Polymères contenant, sur leur masse totale
i) 0,1 à 100 mol % d'au moins un élément de structure de formule III et/ou IIIa 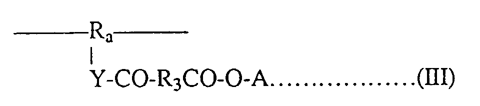 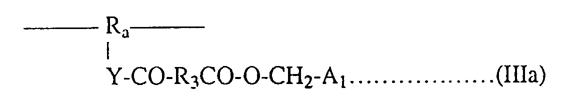 dans lesquelles Rₐ représente le radical d'un groupe apte à une polymérisation radicalaire et R₃, A, A₁ et Y ont les significations indiquées ci-dessus,
ii) 99,9 à 0 mol % d'au moins un élément de structure différent de ceux de formules III et IIa, d'une oléfine apte à une polymérisation radicalaire, et
iii) 80 à 0 mol % d'au moins un élément de structure d'une dioléfine apte à une polymérisation radicalaire, toutes les indications de mol % se complétant à 100 %.

36. Polymères selon la revendication 35, caractérisés en ce que R contient deux à douze atomes de carbone.

37. Polymères selon la revendication 35, caractérisés en ce que Rₐ représente un groupe trivalent éthanetriyle, phénylène-éthylène-, phénylèneméthyl-éthylène- ou éthène-triyle.

38. Polymères selon la revendication 37, caractérisés en ce que les groupes éthane-triyle sont non substitués ou substitués par des groupes alkyle, et dans ce cas, contiennent jusqu'à douze atomes de carbone.

39. Polymères selon la revendication 37, caractérisés en ce que R représente un groupe éthanetriyle, propane-1,2,3-triyle, butane-1,2,4-triyle, pentane-1,2,5-triyle, hexane-1,2,6-triyle, heptane-1,2,7-triyle, octane-1,2,8-triyle, -CH₂-CH(C₆H₄-)- ou -CH₂-CH(C₆H₄CH₂-)-.

40. Polymères selon la revendication 37, caractérisés en ce que le groupe éthène-triyle répond à la formule -HC=-C(CₘH₂ₘ-)- ou -(alkyle en C₁-C₉)C=C(CₘH2ₘ-)- dans laquelle m est un nombre entier allant de 1 à 10 et le nombre total des atomes de carbone est de 3 à 12.

41. Polymères selon la revendication 40, caractérisés en ce que R représente HC=C(CH₂-).

42. Polymères selon la revendication 35, caractérisés en ce que Rₐ représente un groupe de formule -CH₂-CH(CH₂-)-, -CH₂-CH(C₆H₄-)-, -CH₂-CH(C₆H₄CH₂-)-, -HC=C(CH₂-)- ou -(R₁)CH-C(R₂)=, dans laquelle R₁ représente H ou un groupe alkyle en C₁-C₆ et R₂ représente H, un groupe alkyle en C₁-C₆ ou phényle.

43. Polymères selon la revendication 35, caractérisés en ce que le composant i) est contenu en quantité de 0,5 à 100 mol %.

44. Polymères selon la revendication 35, caractérisés en ce que le composant ii) est contenu en quantité de 99,5 à 0,5 mol %.

45. Polymères selon la revendication 35, caractérisés en ce que le composant iii) est contenu en quantité de 70 à 0,01 mol %.

46. Polymères selon la revendication 35, caractérisés en ce que les éléments de structure du composant i) répondent aux formules IV et/ou IVa 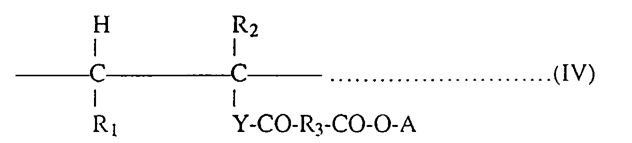 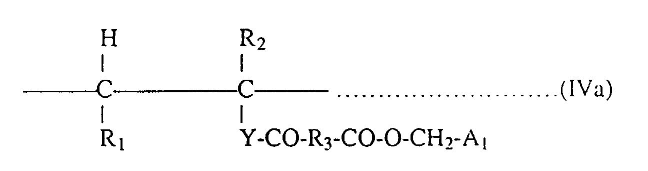 dans lesquelles R₁ représente H ou un groupe alkyle en C₁-C₆ et R₂ représente H, un groupe alkyle en C₁-C₆ ou phényle, et A, A₁, R₃ et Y ont les significations indiquées dans la revendication 35.

47. Polymères selon la revendication 46, caractérisés en ce que R₁ représente H et R₂ représente H, un groupe méthyle, éthyle, n-propyle ou n-butyle.

48. Polymères selon la revendication 46, caractérisés en ce que R₁ et R₂ représentent tous deux H.

49. Polymères selon la revendication 35, caractérisés en ce que les oléfines monomère dont dérivent les éléments de structure du composant ii) consistent en l'éthylène non substitué ou substitué par des halogènes, des groupes -OH, -CN, pyrrolidonyle, alkyle en C₁-C₁₂, phényle, (alkyle en C₁-C₄)phènyle, (alcoxy en C₁-C₄)phényle, halogénophényle, hydroxyphényle, (alkyle en C₁-C₄)hydroxyphényle, (alcoxy en C₁-C₄)hydroxyphényle, chloro- ou bromo-hydroxyphényle, alcoxy en C₁-C₄, phénoxy, (alkyle en C₁-C₄)phénoxy, benzyle, benzyloxy, -COO-M^{⊕}, -COOR₄, -COOCH₂CH(OH)CH₂OH, -COBR₅-OH, -COO-(R₆R₇SiO)ₙ- SiR₆R₆R₈, -COBR₅-O-(R₆R₇SiO)ₙ-SiR₆R₇R₈, CONH₂, -CONH(alkyle en C₁-C₆), -CON(alkyle (en C₁-C₆)₂ ou -OCO-R₄, dans lesquels M^{⊕} représente H^{⊕}, un cation de métal alcalin ou un cation ammonium, R₄ représente un groupe alkyle en C₁-C₁₈, cycloalkyle en C₅-C₇, (alkyle en C₁C₁₂)-cycloalkyle en C₅-C₇, phényle (alkyle en C₁-C₁₂)phényle, benzyle ou (alkyle en C₁-C₁₂)benzyle, R₅ représente un groupe alkylène à chaîne droite ou ramifiée en C₂-C₁₈, un groupe poly-(oxaalkylène en C₂-C₆) contenant deux à six motifs oxaalkylène, un groupe cycloalkylène en C₅-C₈, phénylène, benzylène ou xylylène, B représente -O-, -N(alkyle en C₁-C₆)- ou -NH-, R₆, R₇ et R₈ représentent chacun, indépendamment les uns des autres, un groupe alkyle en C₁-C₆ ou alcoxy en C₁-C₆ et n est un nombre allant de 1 à 30.

50. Polymères selon la revendication 49, caractérisés en ce que R₄ représente un groupe alkyle en C₁-C₆.

51. Polymères selon la revendication 49, caractérisés en ce que R₅ représente un groupe alkylène en C₂-C₆.

52. Polymères selon la revendication 49, caractérisés en ce que R₆, R₇ et R₈ représentent des groupes méthyle, éthyle, méthoxy ou éthoxy.

53. Polymères selon la revendication 49, caractérisés en ce que M^{⊕} est un cation ammonium NH₄O ou le cation d'une amine primaire en C₁-C₁₂, d'une amine secondaire en C₂-C₁₈, d'une amine tertiaire en C₃-C₂₄, ou bien un ion ammonium quaternaire en C₄-C₃₀, de préférence en C₄-C₂₀.

54. Polymères selon la revendication 49, caractérisés en ce que le composant ii) consiste en motifs de structure de formule V 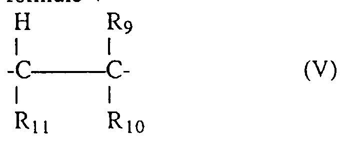 dans laquelle R₁₁ représente H, un groupe alkyle en C₁-C₆, -COOR₂₀ ou -COO^{⊕}M^{⊕}, R₉ représente H, F, Cl, CN ou un groupe alkyle en C₁-C₆, R₁₀ représente H, F, Cl, CN, un groupe pyrrolidonyle, R₁₂ représente O-, un groupe alkyle en C₁-C₁₂, un groupe -OH, -COO-M^{⊕}, -COOR₄,-COOCH₂CH(OH)CH₂OH, -CONH₂, -CONH(alkyle en C₁-C₄), -CON(alkyle en C₁-C₄)₂, -COBR₅-OH, -OCO-R₄, -COO-(R₆R₇SiO)ₙ-SiR₆R₇R₈, -COBR₅-O-(R₆R₇SiO)ₙ-SiR₆R₇R₈, un groupe phényle ou phényle substitué par -OH et/ou par un ou deux groupes méthyle, méthoxy, et/ou un ou deux atomes de Cl ou Br, M^{⊕} représente H^{⊕}, un cation de métal alcalin ou un cation ammonium, R₄ représente un groupe alkyle en C₁-C₁₈, cycloalkyle en C₅-C₇, (alkyle en C₁-C₁₂)-cycloalkyle en C₅-C₇, phényle, (alkyle en C₁-C₁₂)-phényle, benzyle ou (alkyle en C₁-C₁₂)-benzyle, R₁₂ représente un groupe alkylène à chaîne droite ou ramifiée en C₂-C₁₈, un groupe poly-(oxaalkylène en C₂-C₆) contenant deux à six motifs oxaalkylène, un groupe cycloalkylène en C₅-C₈, phénylène, benzylène ou xylylène, B représente -O-, -N(alkyle en C₁-C₄)- ou -NH-, et R₆, R₇ et R8 représentent chacun un groupe méthyle, éthyle, méthoxy ou éthoxy, n est égal à 0 ou représente un nombre allant de 1 à 20, de préférence de 1 à 12.

55. Polymères selon la revendication 54, caractérisés en ce que R₁₁ représente H.

56. Polymères selon la revendication 54, caractérisés en ce que R₉ représente H, Cl ou un groupe alkyle en C₁-C₄.

57. Polymères selon la revendication 54, caractérisés en ce que R₁₁ représente H, R₉ représente H, F, Cl, un groupe méthyle ou éthyle, R₁₀ représente un groupe pyrrolidonyle, -F, -Cl, -CN, -OH, alkyle en C₁-C₄, alcoxy en C₁-C₆, -COO-alkyle en C₁-C₆, -COO-R₅-OH, -COOM^{⊕}, -OOC-alkyle en C₁-C₆, -COOCH₂CH (OH)CH₂OH, -CONH₂, -CONH(alkyle en C₁-C₄), -CON(alkyle en C₁-C₄)₂,-COO-(R₆R₇SiO)ₙ-SiR₆R₇R₈, -COBR₅-O-(R₆R₇SiO)ₙ-SiR₆R₇R₈, phényle, méthylphényle, diméthylphényle, chlorophényle, dichlorophényle, dibromophényle, méthoxyphényle, diméthoxyphényle, hydroxyphényle, M^{⊕} représentant un groupe trialkyl-ammonium contenant un à quatre atomes de carbone dans les groupes alkyle, R₅ représente un groupe alkylène en C₂-C₆, R₆, R₇ et R₈ représentent des groupes méthyle, éthyle, méthoxy ou éthoxy et n est égal à 0 ou représente un nombre allant de 1 à 12.

58. Polymères selon la revendication 54, caractérisés en ce que, dans la formule V, R₁₁ représente H, R₉ représente H ou un groupe méthyle et R₁₀ représente un groupe pyrridonyle, -CN, -COOH, -COO-C_{y}H_{2y}-OH, y allant de 2 à 6, -CON (CH₃)₂, -COO-CH₂CH(OH)CH₂ et -COO-CH₂CH₂-O-[Si(OCH₃)₂-O]ₙ-Si (OCH₃)₃ ou -COO-CH₂CH₂-O-[Si(OC₂H₅)₂-O]ₙ,-Si(OC₂H₅)₃, n allant de 1 à 8 et de préférence de 2 à 6.

59. Polymères selon la revendication 35, caractérisés en ce que le composant iii) consiste en motifs de structure de butadiène, d'isoprène et de chloroprène.

60. Polymères selon la revendication 35, caractérisés en ce que le composant iii) consiste en motifs de structure dérivés de diacrylates ou de diméthacrylates de diols ou de diacryl- ou diméthacryl-amides de diamines.

61. Polymères selon la revendication 60, caractérisés en ce que les diols et diamines répondent à la formule HY-CₓH₂ₓ-YH dans laquelle x est un nombre allant de 2 à 12, et Y représente -O-, -NH- ou -N(alkyle en C₁-C₄)- ou bien consistent en polyoxaalkylènediols de formule HO-(CₘH₂ₘO)_{y}-H dans laquelle m est un nombre allant de 2 à 6 et y un nombre allant de 2 à 20.

62. Lentille de contact contenant un polymère selon revendication 35.

63. Lentille de contact consistant en un polymère selon la revendication 35.

64. Lentille de contact portant une couche extérieure d'un polymère selon la revendication 35.

65. Polymères selon la revendication 35, caractérisés en ce qu'ils contiennent, en tant que quatrième composant iiii), en proportions allant jusqu'à 10 mol %, des radicaux de composés à insaturation éthylénique trifonctionnels, les indications de mol % se complétant à 100 %.

66. Utilisation des polymères hydrosolubles selon la revendication 35 en tant qu'agents épaississants et agents tensio-actifs.

67. Utilisation des polymères selon la revendication 35, en tant que véhicules pour des substances actives dont on demande une libération contrôlée.

68. Utilisation des polymères selon la revendication 35 pour la fabrication, la modification ou le nettoyage de lentilles de contact.
